# EUROPÄISCHE PATENTSCHRIFT

(11) **EP 2 091 911 B1**
(45) Veröffentlichungstag und Bekanntmachung des Hinweises auf die Patenterteilung: **29.04.2015**
(21) Anmeldenummer: 07822199.1
(22) Anmeldetag: 05.11.2007
(51) Int. Cl.: C07C 263/04, C07C 265/14, C07C 269/04

(54) **VERFAHREN ZUR KONTINUIERLICHEN HERSTELLUNG VON (CYCLO)ALIPHATISCHEN DIISOCYANATEN**
PROCESS FOR THE CONTINUOUS PREPARATION OF (CYCLO)ALIPHATIC DIISOCYANATES
PROCÉDÉ DE FABRICATION EN CONTINU DE DIISOCYANATES (CYCLO)ALIPHATIQUES

(30) Priorität: 23.12.2006 DE 102006061475
(43) Veröffentlichungstag der Anmeldung: 26.08.2009
(73) Patentinhaber: Evonik Degussa GmbH, 45128 Essen (DE)
(72) Erfinder: MICHALCZAK, Hans-Werner, 44651 Herne (DE); KOHLSTRUK, Stephan, 48249 Dülmen (DE); KRECZINSKI, Manfred, 44652 Herne (DE); GRUND, Gerda, 48653 Coesfeld (DE); LOMÖLDER, Rainer, 48153 Münster (DE)
(86) Internationale Anmeldenummer: PCT/EP2007/061870
(87) Internationale Veröffentlichungsnummer: WO 2008/077672

(56) Entgegenhaltungen:
- EP-A- 0 355 443
- EP-A- 1 634 868
- DE-A1- 2 836 511

## Beschreibung

Die Erfindung betrifft ein mehrstufiges Verfahren zur kontinuierlichen phosgenfreien Herstellung von (cyclo)aliphatischen Diisocyanaten, das die Umwandlung von (cyclo)aliphatischen Diaminen in die entsprechenden (Cyclo)alkylenbiscarbamate und die thermische Spaltung letzterer in die (Cyclo)alkylendiisocyanate und Alkohol umfasst.

Diisocyanate sind wertvolle chemische Verbindungen, die nach dem Prinzip des Diisocyanat-Polyadditionsverfahrens den gezielten Aufbau von Polymeren erlauben, welche als Polycarbamate oder Polyharnstoffe in Schaumstoffen, Elastomeren, Thermoplasten, Fasern, lichtbeständigen Polycarbamatbeschichtungen oder Klebstoffen vielfältigen industriellen Einsatz finden.

Der synthetische Zugang zu Isocyanaten kann über eine Reihe unterschiedlicher Routen erfolgen. Älteste und auch heute noch vorherrschende Variante zur großtechnischen Herstellung von Isocyanaten ist die Phosgenierung der entsprechenden Amine unter Verwendung von korrosivem, sehr giftigem und einen hohen Anteil an Chlor enthaltendem Phosgen, das besonders hohe Anforderungen an seine Handhabung im industriellen Maßstab stellt.

Es gibt mehrere Verfahren, die Verwendung von Phosgen zur Herstellung von Isocyanaten in technischen Größenordnungen zu umgehen. Der Begriff phosgenfreies Verfahren wird häufig im Zusammenhang mit der Überführung von Aminen in Isocyanate unter Einsatz alternativer Carbonylierungsmittel, z.B. Harnstoff oder Dialkylcarbonat, benutzt (EP 18 586, EP 355 443, US 4,268,683, EP 990 644).

Grundlage der so genannten Harnstoffroute ist die Harnstoff-vermittelte Überführung von Diaminen in Diisocyanate über einen zweistufigen Prozess. Im ersten Prozessschritt wird ein Diamin mit Alkohol, in Gegenwart von Harnstoff oder Harnstoff-Äquivalenten (z. B. Alkylcarbonate, Alkylcarbamate) zu einem Biscarbamat umgesetzt, welches üblicherweise eine Zwischenreinigungstufe durchläuft und dann im zweiten Prozessschritt thermisch in Diisocyanat und Alkohol gespalten wird (EP 126 299, EP 126 300, EP 355 443, US 4,713,476, US 5,386,053). Alternativ kann der eigentlichen Biscarbamatbildung auch die separate Herstellung eines Bisharnstoffs durch gezielte Umsetzung des Diamins mit Harnstoff vorgeschaltet sein (EP 568 782). Denkbar ist auch eine zweistufige Sequenz aus partieller Umsetzung von Harnstoff mit Alkohol im ersten und anschließender Zudosierung und Carbamatisierung des Diamins im zweiten Schritt (EP 657 420).

Die thermische Spaltung von (cyclo)aliphatischen Biscarbamaten kann in der Gas- oder in der Flüssigphase, mit oder ohne Lösungsmittel und mit oder ohne Katalysatoren erfolgen. So werden in der EP 126 299 und in der EP 126 300 Verfahren zur Herstellung von Hexamethylendiisocyanat bzw. Isophorondiisocyanat durch Spaltung der entsprechenden Biscarbamate in der Gasphase im Rohrreaktor in Gegenwart von metallischen Füllkörpern bei 410 °C beschrieben. Abgesehen davon, dass solch hohe Temperaturen nur noch mit aufwendiger Technologie einzustellen sind, ist das Verfahren mit dem Nachteil behaftet, dass bei dieser Temperatur bereits teilweise Zersetzung der Reaktionsprodukte stattfindet, verbunden mit Ablagerungen auf den Füllkörpern und einem Zuwachsen des Rohrreaktors, so dass das Verfahren wegen der kurzen Standzeit für eine technische Produktion wenig geeignet ist.

Es hat daher nicht an Vorschlägen gefehlt, durch chemische und verfahrenstechnische Maßnahmen Ausbeuteverbesserungen zu erzielen und die unerwünschte Nebenproduktbildung einzuschränken. So wird in einer Reihe von Dokumenten der Einsatz von Katalysatoren beschrieben, die die Spaltreaktion der Biscarbamate beschleunigen (DE 1 022 222, US 3,919,279, DE 2 635 490). Tatsächlich gelingt es in Gegenwart geeigneter Katalysatoren - hierbei handelt es sich um eine Vielzahl basischer, saurer sowie metallorganischer Verbindungen - durchaus, die Isocyanatausbeute im Vergleich zur unkatalysierten Variante zu steigern. Die Bildung unerwünschter Nebenprodukte kann jedoch auch durch die Anwesenheit eines Katalysators nicht vermieden werden. Dasselbe gilt für die zusätzliche Verwendung von inerten Lösungsmitteln, wie sie ebenfalls in der US 3,919,279 und DE 2 635 490 empfohlen werden, um eine gleichmäßige Verteilung der zugeführten Wärme und des Katalysators im Reaktionsmedium zu gewährleisten. Grundsätzlich hat die Verwendung von unter Rückfluss siedenden Lösungsmitteln jedoch eine Reduzierung der Raum/Zeit-Ausbeute an Isocyanaten zur Folge und ist darüber hinaus mit dem Nachteil eines zusätzlichen hohen Energieaufwands behaftet.

In der EP 54 817 angeführte Beispiele zur thermisch geführten katalysierten Spaltung von Monocarbamaten beschreiben die Teilausschleusung des Reaktionsgemisches zur Abtrennung der im Zuge der Carbamatspaltung entstehenden verharzenden Nebenprodukte. Diese Prozedur dient der Vermeidung von Belegungen und Verstopfungen in Reaktoren und Aufarbeitungseinrichtungen. Hinweise, die auf eine Ausbeute steigernde Verwertung der Teilausschleusung hindeuten, gibt es nicht. Die EP 61 013 beschreibt einen ähnlichen Lösungsansatz, wobei die Thermolyse in diesem Fall in Gegenwart von Lösungsmitteln durchgeführt wird, deren Aufgabe offenbar in einer besseren Aufnahme der schwerflüchtigen Nebenprodukte besteht. Auch hier wird die Teilausschleusung nicht im Sinne einer Ausbeuteoptimierung verwertet.

Aus der EP 355 443 ist nun bekannt, dass eine Ausbeutesteigerung erzielt werden kann, wenn die während der Spaltung von Biscarbamaten im Spaltreaktor entstehenden höhermolekularen, verwertbaren und nicht verwertbaren Nebenprodukte zur Gewährleistung einer störungsfreien und selektiven Reaktion möglichst kontinuierlich aus dem Reaktor ausgeschleust werden und anschließend in Gegenwart von Alkohol zum großen Teil umgesetzt und dann in die (Cyclo)alkylenbiscarbamatsynthese zurückgeführt werden. Die beschriebene Verfahrensweise ist mit einem hohen Energieaufwand verbunden, da die Abtrennung nicht verwertbarer Nebenprodukte aus dem Austrag der (Cyclo)alkylenbiscarbamatsynthese destillativ erfolgt, wobei das gesamte Biscarbamat verdampft werden muss. Im Unterschied zur EP 355 443 wird der Biscarbamataustrag beim Verfahren der EP 566 925 in zwei Teilströme aufgeteilt, von denen nur einer destillativ von seinen hochsiedenden, nicht verwertbaren Nebenprodukten befreit wird, bevor die vereinigten Biscarbamatströme der Deblockierungsreaktion im Spaltreaktor zugeführt werden. Zudem wird die kontinuierliche Spaltreaktorausschleusung bei der EP 566 925 direkt, d. h. ohne Recarbamatisierungsschritt, in die (Cyclo)alkylenbiscarbamat-synthese zurückgeführt.

Die Herstellung von (cyclo)aliphatischen Biscarbamaten in einer Eintopfreaktion aus Diamin, Harnstoff und Alkohol unter gleichzeitiger Abtrennung von Ammoniak ist aus der EP 18 568 bekannt. Die Lehre der EP 18 568 wurde weiterentwickelt und ist in EP 126 299, EP 126 300, EP 355 443, EP 566 925 beschrieben. Nachteil der dort vorgenommenen simultanen Umsetzung von Diamin, Harnstoff und Alkohol ist die durch unvermeidbar ablaufende Nebenreaktionen verminderte Selektivität durch zwangsläufig und in größerer Menge gebildeten Nebenprodukte, die vor der thermischen Deblockierung der Biscarbamate abgetrennt werden müssen. Die EP 568 782 beansprucht daher ein kontinuierliches Verfahren zur Herstellung von (cyclo)aliphatischen Diisocyanaten, welches im Wesentlichen drei Hauptschritte umfasst, von denen der erste die Bildung von (Cyclo)alkylenbisharnstoffen, der zweite die Bildung von (Cyclo)alkylenbiscarbamaten aus den (Cyclo)alkylenbisharnstoffen und der dritte die Spaltung der (Cyclo)alkylenbiscarbamate in flüssiger Phase zu den gewünschten (cyclo)aliphatischen Diisocyanaten beschreibt - d. h., die Herstellung des (Cyclo)alkylenbiscarbamats erfolgt in zwei getrennten Stufen. Nach der Lehre der EP 568 782 wird der Austrag der Reaktionssequenz aus (Cyclo)alkylenbisharnstoff-Bildung und anschließender (Cyclo)alkylenbiscarbamatsynthese zunächst destillativ von Leicht- und Mittelsiedern wie Alkoholen, Carbamaten und Carbonaten befreit und der Hochsieder im Biscarbamat danach durch Kurzwegverdampfung abgetrennt. Das (Cyclo)alkylenbiscarbamat wird thermisch deblockiert und ein Teil des Spaltsumpfes wird kontinuierlich ausgeschleust, mit Alkohol recarbamatisiert und wieder in die (Cyclo)alkylenbiscarbamatsynthese-Stufe zurückgeführt.

Beschrieben ist auch, in der EP 1 634 868 dass es bei Einsatz von (cyclo)aliphatischen Diaminen neben der einstufigen Synthese von (cyclo)aliphatischen Biscarbamaten aus (cyclo)aliphatischen Diaminen, Alkohol und Harnstoff vorteilhaft ist, die (cyclo)aliphatischen Biscarbamate durch zweistufige und somit über (cyclo)aliphatischen Bisharnstoff verlaufende Umsetzung von (cyclo)aliphatischen Diaminen mit Alkohol und Harnstoff herzustellen, sie von Leicht- und Mittelsiedern zu befreien, die so gereinigten (cyclo)aliphatischen Biscarbamate unter Freisetzung des gewünschten (cyclo)aliphatischen Diisocyanats thermisch zu spalten, einen Teil des Spaltsumpfes aus der Spaltapparatur kontinuierlich auszuschleusen und mit Alkohol zu recarbamatisieren, hieraus Hochsiederkomponenten abzutrennen und das so gereinigte Recarbamatisat in den Prozess zu recyclieren, oder auch, aus dem aus der Spaltapparatur kontinuierlich ausgeschleusten Teil des Spaltsumpfes, die Hochsiederkomponenten direkt abzutrennen, das gewonnene Destillat mit Alkohol zu recarbamatisieren und das Recarbamatisat in den Prozess zu recyclieren. Es hat sich herausgestellt, dass durch diese Verfahrensschritte zum einen eine vergleichsweise niedrige stationäre Konzentration an Hochsiederkomponenten über die gesamte Sequenz Biscarbamat-Synthese, Biscarbamat-Reinigung und Biscarbamat-Spaltung realisiert wird, so dass Ablagerungen, die insbesondere durch die von Natur aus hochviskosen Hochsiederkomponenten begünstigt werden, weitgehend vermieden werden können und auch langfristig eine gute Anlagenverfügbarkeit und eine gute Verfahrensausbeute gewährleistet werden. Zum anderen hat die der thermischen Spaltreaktion nachgeschaltete Sequenz aus Recarbamatisierung und Hochsiederabtrennung bzw. Hochsiederabtrennung mit nachfolgender Recarbamatisierung des Destillates den Vorteil, dass im Vergleich zur üblichen Vorgehensweise, bei der der Hochsieder vor der Biscarbamatspaltung abgetrennt wird, die in die Dampfphase zu überführende Biscarbamatmenge signifikant verringert ist, wodurch Investment- und Energiekosten eingespart werden können.

Des Weiteren ist in der EP 1 582 680 beschrieben, dass es bei Einsatz von (cyclo)aliphatischen Diaminen vorteilhaft ist, die (cyclo)aliphatischen Biscarbamate nach ihrer Synthese durch Umsetzung von (cyclo)aliphatischen Diaminen mit Harnstoff und Alkohol und/oder Harnstoffderivate von Leicht- und Mittelsiedern zu befreien, die so gereinigten (cyclo)aliphatischen Biscarbamate unter Freisetzung des gewünschten (cyclo)aliphatischen Diisocyanats thermisch zu spalten, einen Teil des Spaltsumpfes aus der Spaltapparatur kontinuierlich auszuschleusen und hieraus Hochsiederkomponenten abzutrennen, und die so gereinigte Ausschleusung mit Alkohol zu recarbamatisieren und in den Prozess zu recyclieren. Es hat sich herausgestellt, dass auf diese Weise zum einen eine vergleichsweise niedrige stationäre Konzentration an Hochsiederkomponenten über die gesamte Sequenz Biscarbamat-Synthese, Biscarbamat-Reinigung und Biscarbamat-Spaltung realisiert wird, so dass Ablagerungen, die insbesondere durch die von Natur aus hochviskosen Hochsiederkomponenten begünstigt werden, weitgehend vermieden werden können und auch langfristig eine gute Anlagenverfügbarkeit und eine gute Verfahrensausbeute gewährleistet sind. Zum anderen hat die der thermischen Spaltreaktion nachgeschaltete Hochsiederabtrennung den Vorteil, dass im Vergleich zur üblichen Vorgehensweise, bei der der Hochsieder vor der Biscarbamatspaltung abgetrennt wird, die in die Dampfphase zu überführende Biscarbamatmenge signifikant verringert ist, wodurch Investment- und Energiekosten eingespart werden können.

Die heute vorherrschende Handelsform großtechnisch hergestellten Harnstoffs sind Prills, d. h. kleine Kügelchen mit einem Durchmesser von 1 - 3 mm. Kristalliner Harnstoff neigt auch bei sehr niedrigen Wassergehalten von < 0,1 % so stark zum Verbacken, dass er für eine lose Lagerung in großen Mengen nicht in Frage kommt. Eine Verbesserung der Lagereigenschaften von Harnstoff-Prills, die beispielsweise bei Silolagerung von großen Mengen notwendig erscheint, wird durch eine nachträgliche Oberflächenbehandlung der Prills mit Puderstoffen wie beispielsweise Talkum, Bentonite, Kieselgur, Diatomeen oder andere silikatische Stoffe oder durch Schwefel und auch durch Aufdüsen von geringen Mengen Öl erzielt. ,

Die Harnstoffindustrie setzt heute bevorzugt Formaldehyd bis zu 0,6 Gew.-% (Ullmann's Encyclopedia of Industrial Chemistry, Release 2006, 7th Edition) der Harnstoffschmelze vordem Verprillen zu um die Stabilität der Prills zu erhöhen. Diese Maßnahme dient zur Vorbeugung gegen Zerfall und Verbackung beim Transport und zur Verbesserung der Lagerstabilität.

Harnstoff aus einer mit Formaldehyd (auch Paraformaldehyd) vor dem Prillen oder Granulieren behandelten Harnstoffschmelze und mit Formaldehyd (auch Paraformaldehyd) oberflächenbehandelter Harnstoff, ebenfalls eine industriell praktizierte Maßnahme zur Verbesserung der Lagereigenschaft der Prills, führt sowohl bei einstufigen, zweistufigen und auch alternativ nach mehrstufigen Verfahren zur Herstellung von (cyclo)aliphatischen Biscarbamaten als auch bei der sich anschließenden thermischen Spaltung der (cyclo)aliphatischen Biscarbamate zu (cyclo)aliphatischen Diisocyanaten zur Bildung von unerwünschten Nebenprodukten.

Die gebildeten Nebenprodukte führen in der kontinuierlich betriebenen ein- oder mehrstufigen Biscarbamatsynthese nach kurzer Laufzeit nicht nur zu unerwünschten Anbackungen in den Apparaten mit der Folge von relativ kurzen Produktionszeiträumen mit nachfolgenden aufwendigen Reinigungsprozeduren sondern sind in den bekannten und in der Literatur ausführlich beschriebenen verschiedenen Stufen der destillativen Aufarbeitung des Roh-Biscarbamats nur unzureichend abtrennbar.

In den Aggregaten zur thermischen Spaltung des (cyclo)aliphatischen Biscarbamats zum (cyclo)aliphatischen Diisocyanat führen sowohl die nicht quantitativ abgetrennten Nebenprodukte aus der Biscarbamatstufe selbst, als auch ein daraus neu generiertes Nebenproduktspektrum zusätzlich zu Anbackungen und damit zur Reduzierung der Anlagenverfügbarkeit durch aufwendige Reinigungsprozeduren.

Der Erfindung liegt die Aufgabe zugrunde, ein verbessertes Verfahren zur Herstellung von (cyclo)aliphatischen Diisocyanaten bereitzustellen, das die oben genannten Nachteile vermeidet.

Überraschenderweise wurde die Aufgabe dadurch gelöst, dass zur kontinuierlichen Herstellung von (cyclo)aliphatischen Diisocyanaten, durch Umsetzung von (cyclo)aliphatischen Diaminen mit Harnstoff und Alkoholen zu (cyclo)aliphatischen Biscarbamaten und anschließender thermischer Spaltung der (cyclo)aliphatischen Biscarbamate zu (cyclo)aliphatischen Diisocyanaten zur Bildung der (cyclo)aliphatischen Biscarbamate nach einstufigen, zweistufigen und auch alternativ nach mehrstufigen Verfahren, unkonditionierter Harnstoff, unabhängig von der Darreichungsform (Prills, Granulat, Kristalle, Schmelze, Lösung) eingesetzt wird. Unkonditionierter Harnstoff ist weder oberflächenbehandelt noch sind zur Schmelze vor dem Prillen oder Granulieren Zuschlagstoffe und/oder Formaldehyd zugesetzt worden.

Der erfindungsgemäß eingesetzte Harnstoff ist unkonditioniert das heißt er darf weder mit anorganischen Substanzen oberflächenbehandelt sein, wie beispielsweise mit Talkum, Bentonite, Kieselgur, Diatomeen, Kaolin oder anderen silikatischen Stoffen, die auch als Antibackmittel Anwendung finden, und/oder aus einer mit Formaldehyd (auch Paraformaldehyd) behandelten Harnstoffschmelze stammen und/oder mit Formaldehyd (auch Paraformaldehyd) oberflächenbehandelt sein. Im Allgemeinen beträgt die maximale Formadehydkonzentration (auch Paraformaldehyd) des eingesetzten Harnstoffs oder der eingesetzten Harnstoffäquivalente 0,01 bis 0,10 Gew.-%, vorzugsweise 0,001 bis 0,01 Gew.-% und besonders bevorzugt weniger als 0,001 Gew-%.

Das erfindungsgemäße Verfahren hat den Vorteil, dass eine quantitative Abtrennung des üblicherweise generierten Nebenproduktspektrums mit den beschriebenen Destillations- und Rektifikationseinrichtungen zur Reinigung der (cyclo)aliphatischen Diisocyanate unterbleiben kann und führt zu Diisocyanatqualitäten deren anwendungstechnisches Eigenschaftsprofil einen Einsatz in weitere Veredelungsstufen ohne zusätzliche Aufarbeitungsschritte möglich macht.

Die Abtrennung des in der Biscarbamatstufe aus Formaldehyd und/oder aus formaldehydhaltigen Komponenten des eingesetzten unkonditionierten Harnstoffs bei der Reaktion mit (cyclo)aliphatischen Diaminen in Gegenwart von Alkoholen generierten Nebenproduktspektrums ist nicht erforderlich und der Einsatz von zusätzlichen Apparaten ist nicht nötig. Aufgrund der Vermeidung von Nebenprodukten in der Biscarbamatstufe wird zusätzlich anschließend ein weiteres Nebenproduktspektrum in der thermischen Spaltung der (cyclo)aliphatischen Biscarbamate zu (cyclo)aliphatischen Diisocyanaten vermieden, Auch in dieser Stufe der destillativen Reinigung der Diisocyanate ist der Einsatz zusätzlicher Apparate zur Erzielung der gewünschten Diisocyanatreinheiten überflüssig.

Ein zusätzliches Investment verbunden mit einer signifikanten Minderung der Gesamtverfahrensausbeute durch Diaminverluste, hervorgerufen durch die Nebenproduktbildung, die zuzüglich infolge der verursachenden Verbackungen in verschiedenen Apparateteilen durch aufwendige Reigungsprozeduren die Anlagenverfügbarkeit reduzieren, konnte überraschenderweise durch den erfindungsgemäßen Einsatz von unkonditionierten Harnstoffen völlig vermieden und somit die Wirtschaftlichkeit des Verfahrens gesteigert werden.

Gegenstand der Erfindung ist ein Verfahren zur kontinuierlichen Herstellung von (cyclo)aliphatischen Diisocyanaten durch Umsetzung von mindestens einem (cyclo)aliphatischen Diamin mit Harnstoff und mindestens einem Alkohol zu (cyclo)aliphatischen Biscarbamaten und anschließender thermischer Spaltung der (cyclo)aliphatischen Biscarbamate zu (cyclo)aliphatischen Diisocyanaten dadurch gekennzeichnet, dass zur Bildung der (cyclo)aliphatischen Biscarbamate unkonditionierter Harnstoff eingesetztwird, wobei der Harnstoff weder mit anorganischen Substanzen oberflächenbehandelt sein darf, und/oder aus einer mit Formaldehyd oder auch Paraformaldehyd behandelten Harnstoffschmelze stammen darf und/oder mit Formaldehyd oder auch Paraformaldehyd oberflächenbehandelt sein darf.

Bevorzugter Gegenstand der Erfindung ist ein mehrstufiges Verfahren zur kontinuierlichen Herstellung von (cyclo)aliphatischen Diisocyanaten durch Umsetzung von mindestens einem (cyclo)aliphatischen Diamin mit Harnstoff und mindestens einem Alkohol zu (cyclo)aliphatischen Biscarbamaten und anschließender thermischer Spaltung der (cyclo)aliphatischen Biscarbamate zu (cyclo)aliphatischen Diisocyanaten dadurch gekennzeichnet, dass zur Bildung der (cyclo)aliphatischen Biscarbamate nach einstufigen, zweistufigen oder alternativ auch mehrstufigen Verfahren, unabhängig von der Darreichungsform (z. B. Prills, Granulat, Kristalle, Schmelze, Lösung), unkonditionierter Harnstoff eingesetztwird.

Die maximale Formaldehydkonzentration (auch Paraformaldeyd) des eingesetzten Harnstoffs oder der eingesetzten Harnstoffäquivalente beträgt 0,01 bis 0,10 Gew.-%, vorzugsweise 0,001 bis 0,01 Gew.-% und besonders bevorzugt weniger als 0,001 Gew.-%.

Gegenstand der Erfindung ist ein Verfahren zur kontinuierlichen Herstellung von (cyclo)aliphatischen Diisocyanaten der Formel (I)

OCN-R-NCO

wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, durch Umsetzung (cyclo)aliphatischer Diamine mit unkonditioniertem Harnstoff und Alkoholen zu (cyclo)aliphatischen Biscarbamaten und deren thermische Spaltung, das durch die folgenden einzelnen Schritte charakterisiert ist:
a) (cyclo)aliphatische Diamine der Formel (II)

   H₂N-R-NH₂

   wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, mit unkonditioniertem Harnstoff werden in Gegenwart von Alkohol der Formel (III)

   R¹-OH

   wobei R¹ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Dialkycarbonaten, Carbamidsäurealkylestern oder Mischungen aus Dialkylcarbonaten und Carbamidsäureestern und in Abwesenheit oder Gegenwart von Katalysatoren zu (Cyclo)alkylenbisharnstoff der Formel (IV) umgesetzt

   H₂N-OC-HN-R-NH-CO-NH₂

   wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, in einem Destillationsreaktor, unter gleichzeitiger Abtrennung des entstehenden Ammoniaks, wobei die Edukte kontinuierlich auf den obersten Boden aufgegeben werden und das gebildete Ammoniak durch Destillation mit Alkoholbrüden, die im Sumpf eingeführt werden, ausgetrieben wird;
b) in der zweiten Stufe wird die Umsetzung des aus der ersten Stufe a) anfallenden (Cyclo)alkylenbisharnstoffs mit dem in a) als Lösungsmittel eingesetzten Alkohol in einem Druckdestillationsreaktor, unter gleichzeitiger Abtrennung des entstehenden Ammoniaks, zum (Cyclo)alkylenbiscarbamat der Formel (V)

   R¹O-OC-HN-R-NH-CO-OR¹

   durchgeführt;
c) oder optional die Umsetzung (cyclo)aliphatischer Diamine der Formel (II)

   H₂N-R-NH₂

   mit unkonditioniertem Harnstoff in Gegenwart von Alkohol der Formel (III)

   R¹-OH

   in einem Druckdestillationsreaktor einstufig, unter gleichzeitiger Abtrennung des entstehenden Ammoniaks, zum (Cyclo)alkylenbiscarbamat der Formel (V)

   R¹O-OC-HN-R-NH-CO-OR¹

   durchgeführt wird ohne die Schritte a) und b) (R und R¹ entspricht der obigen Definition);
d) aus der erhaltenen Reaktionsmischung aus b) oder optional c) wird der Alkohol, die Dialkylcarbonate und/oder Carbamidsäurealkylester abgetrennt und der Alkohol sowie optional, auch die Dialkylcarbonate und/oder Carbamidsäurealkylester, in die Reaktionsstufe a) oder b) oder optional c) zurückgeführt;
e) die Abtrennung von Ammoniak aus den am Kopf des Druckdestillationsreaktors sowohl aus b) oder optional aus c) anfallenden Brüden und aus dem Alkohol, der durch partielle Kondensation der Brüden aus dem Destillationsreaktor a) oder optional c) gewonnen wird, in einer nachgeschalteten Kolonne zweckmäßigerweise unter dem Druck des Druckdestillationsreaktors durchgeführt wird, wobei der im Sumpf anfallende ammoniakfreie Alkohol in den Sumpf des Destillationsreaktors und/oder in den Sumpf des Druckdestillationsreaktors recycliert wird;
f) das von Leichtsiedern abgereicherte Roh-(cyclo)alkylenbiscarbamat aus d) wird vollständig oder partiell von hochsiedenden Rückständen abgetrennt oder optional wird auf eine Rückstandsabtrennung verzichtet;
g) die über die Schritte d) und optional f) vorbehandelte (Cyclo)alkylenbiscarbamate enthaltende Reaktionsmischung wird in Gegenwart eines Katalysators kontinuierlich und lösemittelfrei bei Temperaturen von 180 bis 280°C, vorzugsweise 200 bis 260 °C, und unter einem Druck von 0,1 bis 200 mbar, vorzugsweise 0,2 bis 100 mbar thermisch so gespalten, dass ein Teil des Reaktionsgemisches von 10 bis 60 Gew.-% bezogen auf den Feed, vorzugsweise 15 bis 45 Gew.-% bezogen auf den Feed, ständig aus dem Sumpf ausgeschleust wird;
h) die Spaltprodukte aus Schritt g) werden durch Rektifikation in ein (cyclo)aliphatisches Rohdiisocyanat und Alkohol getrennt;
i) das (cyclo)aliphatische Rohdiisocyanat wird durch Destillation gereinigt und die (cyclo)aliphatisches Reindiisocyanat enthaltende Fraktion isoliert;
j) die Sumpfausschleusung aus g) wird partiell oder vollständig mit dem Alkohol aus h) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wobei das Molverhältnis von NCO-Gruppen und OH-Gruppen bis zu 1 : 100, vorzugsweise 1 : 20 und besonders bevorzugt 1 : 10 beträgt;
k) die Reaktionsmischung aus j) wird in einen Wertstoff- und einen Abfallstrom aufgetrennt und der an Hochsiederkomponenten reiche Abfallstrom aus dem Prozess ausgeschleust und verworfen;
l) optional wird die Reaktionsmischung aus j) direkt in die (Cyclo)alkylenbiscarbamatstufe b) oder optional c) recycliert;
m) ein Teil der Sumpffraktion der Reindestillation i) wird kontinuierlich ausgeschleust und in die Spaltreaktion g) und/oder in die Carbamatisierungsstufe j) geführt;
n) optional werden die bei der Reindestillation des (cyclo)aliphatischen Rohdiisocyanats anfallenden Kopffraktionen ebenfalls in die Carbamatisierungsstufe j) zurückgeführt;
o) der Wertstoffstrom aus k) wird in Stufe b) oder optional c) und/oder d) und/oder g) zurückgeführt.

Gegenstand der Erfindung ist auch ein mehrstufiges Verfahren zur kontinuierlichen Herstellung von (cyclo)aliphatischen Diisocyanaten der Formel (I)

OCN-R-NCO

wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, durch Umsetzung (cyclo)aliphatischer Diamine mit unkonditioniertem Harnstoff und Alkoholen zu (cyclo)aliphatischen Biscarbamaten und deren thermische Spaltung, wobei
a) (cyclo)aliphatische Diamine der Formel (II)

   H₂N-R-NH₂

   wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, mit unkonditioniertem Harnstoff in Gegenwart von Alkohol der Formel (III)

   R¹-OH

   wobei R¹ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Dialkycarbonaten, Carbamidsäurealkylestern oder Mischungen aus Dialkylcarbonaten und Carbamidsäureestern und in Abwesenheit oder Gegenwart von Katalysatoren zu (Cyclo)alkylenbisharnstoff der Formel (IV)

   H₂N-OC-HN-R-NH-CO-NH₂

   wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, in einem Destillationsreaktor, unter gleichzeitiger Abtrennung des entstehenden Ammoniaks, umgesetzt werden, wobei die Edukte kontinuierlich auf den obersten Boden aufgegeben werden und das gebildete Ammoniak durch Destillation mit Alkoholbrüden, die im Sumpf eingeführt werden, ausgetrieben wird;
b) in der zweiten Stufe die Umsetzung des aus der ersten Stufe a) anfallenden (Cyclo)alkylenbisharnstoffs mit dem in a) als Lösungsmittel eingesetzten Alkohol in einem Druckdestillationsreaktor, unter gleichzeitiger Abtrennung des entstehenden Ammoniaks, zum (Cyclo)alkylenbiscarbamat der Formel (V)

   R¹O-OC-HN-R-NH-CO-OR¹

   durchgeführt wird;
c) oder optional die Umsetzung (cyclo)aliphatischer Diamine der Formel (II)

   H₂N-R-NH₂

   mit unkonditioniertem Harnstoff in Gegenwart von Alkohol der Formel (III)

   R¹-OH

   in einem Druckdestillationsreaktor einstufig, unter gleichzeitiger Abtrennung des entstehenden Ammoniaks, zum (Cyclo)alkylenbiscarbamat der Formel (V)

   R¹O-OC-HN-R-NH-CO-OR¹

   durchgeführt wird ohne die Schritte a) und b) (R und R¹ entspricht der obigen Definition);
d) aus der erhaltenen Reaktionsmischung aus b) oder optional c) der Alkohol, die Dialkylcarbonate und/oder Carbamidsäurealkylester abgetrennt werden und man den Alkohol sowie optional, auch die Dialkylcarbonate und/oder Carbamidsäurealkylester, in die Reaktionsstufe a) oder b) oder optional c) zurückführt;
e) die Abtrennung von Ammoniak aus den am Kopf des Druckdestillationsreaktors sowohl aus b) oder optional aus c) anfallenden Brüden und aus dem Alkohol, der durch partielle Kondensation der Brüden aus dem Destillationsreaktor a) oder optional c) gewonnen wird, in einer nachgeschalteten Kolonne zweckmäßigerweise unter dem Druck des Druckdestillationsreaktors durchführt, wobei der im Sumpf anfallende ammoniakfreie Alkohol in den Sumpf des Destillationsreaktors und/oder in den Sumpf des Druckdestillationsreaktors recycliert wird;
f) das von Leichtsiedern abgereicherte Roh-(cyclo)alkylenbiscarbamat aus d) vollständig oder partiell von hochsiedenden Rückständen abgetrennt oder optional auf eine Rückstandsabtrennung verzichtet wird;
g) die über die Schritte d) und optional f) vorbehandelte (Cyclo)alkylenbiscarbamate enthaltende Reaktionsmischung in Gegenwart eines Katalysators kontinuierlich und lösemittelfrei bei Temperaturen von 180 bis 280°C, vorzugsweise 200 bis 260 °C, und unter einem Druck von 0,1 bis 200 mbar, vorzugsweise 0,2 bis 100 mbar thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig aus dem Sumpf ausgeschleust wird;
h) die Spaltprodukte aus Schritt g) durch Rektifikation in ein (cyclo)aliphatisches Rohdiisocyanat und Alkohol getrennt werden;
i) das (cyclo)aliphatische Rohdiisocyanat durch Destillation gereinigt und die (cyclo)aliphatisches Reindiisocyanat enthaltende Fraktion isoliert wird;
j) die Sumpfausschleusung aus g) partiell oder vollständig mit dem Alkohol aus h) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird und das Molverhältnis von NCO-Gruppen und OH-Gruppen bis zu 1 : 100, vorzugsweise 1 : 20 und besonders bevorzugt 1 : 10 beträgt;
k) die Reaktionsmischung aus j) in einen Wertstoff- und einen Abfallstrom aufgetrennt und der an Hochsiederkomponenten reiche Abfallstrom aus dem Prozess ausgeschleust und verworfen wird;
l) optional die Reaktionsmischung aus j) direkt in die (Cyclo)alkylenbiscarbamatstufe b) oder optional c) recycliert wird;
m) ein Teil der Sumpffraktion der Reindestillation i) kontinuierlich ausgeschleust und in die Spaltreaktion g) und/oder in die Carbamatisierungsstufe j) geführt wird;
n) optional die bei der Reindestillation des (cyclo)aliphatischen Rohdiisocyanats anfallenden Kopffraktionen ebenfalls in die Carbamatisierungsstufe j) zurückgeführt werden;
o) der Wertstoffstrom aus k) in Stufe b) und/oder optional c) und/oder d) und/öder g) zurückgeführt wird.

Nach dem erfindungsgemäßen Verfahren können (cyclo)aliphatische Diisocyanate, das heißt aliphatische und cycloaliphatische, im kontinuierlichen Betrieb problemlos mit sehr guter Ausbeute und hohen Reinheiten hergestellt werden. Vorteilhaft bei dem erfindungsgemäßen Mehrstufenverfahren ist insbesondere die Tatsache, dass bei Einsatz von unkonditioniertem Harnstoff bei gleichzeitigem Einsatz von (cyclo)aliphatischen Diaminen der Formel (II) als Ausgangsmaterialien für die kontinuierliche Diisocyanatsynthese Anbackungen und Ablagerungen, die von konditioniertem Harnstoff hervorgerufen und insbesondere durch die negativen Eigenschaften der hochviskosen Hochsiederkomponenten begünstigt werden, weitgehend vermieden werden können. Außerdem ist der Einsatz von unkonditioniertem Harnstoff als vorteilhaft anzusehen, da, durch die erodierenden Eigenschaften von konditioniertem Harnstoff beispielsweise mit anorganischen Antibackmitteln auftretenden Materialverschleiß und -abtragungen bis hin zur Zerstörung von beweglichen Apparateteilen, vermieden werden kann, und somit auch langfristig eine gute Anlagenverfügbarkeit gewährleistet ist. Weiterhin ist es ein wesentlicher Vorteil, dass, im Gegensatz zu hauptsächlich mit Formaldehyd oder formaldehydhaltigen Komponenten konditioniertem Harnstoff, der in den verschiedenen Verfahrensstufen unerwünschte und nur sehr schwierig abtrennbare Nebenprodukte hervorruft, durch den Einsatz von unkonditioniertem Harnstoff mit hohen Reinheiten hergestellt werden können deren anwendungstechnisches Eigenschaftsprofil einen Einsatz in weitere Veredelungsstufen nicht in Frage stellt.

Im weiteren Text werden die Begriffe Diamin, Alkohol, Bisharnstoff, Biscarbamat und Diisocyanat im Sinne der o. g. Definitionen verwendet.
a) Die Umsetzung von Diamin mit unkonditioniertem Harnstoff wobei die maximale Formaldehydkonzentration (auch Paraformaldeyd) des eingesetzten Harnstoffs 0,01 bis 0,10 Gew.-%, vorzugsweise 0,001 bis 0,01 Gew.-% und besonders bevorzugt < 0,001 Gew.-% betragen darf, zum Bisharnstoff in Gegenwart von Alkohol als Lösungsmittel erfolgt in einem Destillationsreaktor. Die Edukte werden kontinuierlich auf den obersten Boden des Destillationsreaktors aufgegeben und das freigesetzte Ammoniak durch Alkoholbrüden, die im Sumpf des Destillationsreaktors eingeführt werden, ausgetrieben. Das Ammoniak/Alkohol-Gemisch wird, um die Abscheidung von Ammoniumcarbamat zu vermeiden, in einem Kondensator bei Temperaturen von 30 bis 50 °C partiell kondensiert. Aus dem Kondensat wird ammoniakfreier Alkohol durch Destillation in der dem Druckdestillationsreaktor nachgeschalteten Kolonne zurückgewonnen.
   Das Molverhältnis der Edukte Diamin : Harnstoff: Alkohol beträgt 1: 2,0 bis 2,4 : 3 bis 10. Der Destillationsreaktor besitzt mindestens 4 Böden. Die Reaktion wird bei Temperaturen von 100 bis 130 °C und Drücken von 0,7 bis 1,5 bar (abs.) durchgeführt. Die erforderliche Verweilzeit im Destillationsreaktor beträgt 4 bis 10 h, vorzugsweise 6 bis 8 h. Die zum Austreiben des Ammoniaks im Sumpf eingebrachte Alkoholmenge beträgt 0,05 bis 3 kg/kg, vorzugsweise 0,1 bis 1 kg/kg Bisharnstoff, wobei die so eingebrachte Alkoholmenge zusammen mit dem gebildeten Ammoniak am Kopf abgezogen, nach partieller Kondensation in einer Alkoholrückgewinnungskolonne von restlichem Ammoniak befreit und in den Sumpf zurückgeführt wird.
   Um möglichst vollständige Umsetzung des Harnstoffs zum Bisharnstoff zu erreichen, ist die Reaktionstemperatur auf maximal 130 °C begrenzt. Die aus der gewünschten Reaktionstemperatur, der Art und dem Verhältnis der Edukte sich ergebende Reaktionsgeschwindigkeit bestimmt die Verweilzeit und damit die Dimensionierung des Destillationsreaktors.
b) Der im Sumpf des Destillationsreaktors anfallende, in Alkohol gelöste Roh-Bisharnstoff wird kontinuierlich in einen zweiten Reaktor gefahren, in dem die Umsetzung zum Biscarbamat bei erhöhter Temperatur und erhöhtem Druck erfolgt, wobei wiederum Ammoniak freigesetzt wird, das aus Gründen des chemischen Gleichgewichts aus dem Reaktionsgemisch entfernt werden muss. Die weitere Umsetzung des Roh-Bisharnstoff aus a) erfolgt vorzugsweise in einem Druckdestillationsreaktor und bei einem molaren Verhältnis von Bisharnstoff zu Alkohol von 1 : 5 bis 12. Dabei wird der Stoffstrom aus a) vorzugsweise kontinuierlich auf den obersten Boden des Druckdestillationsreaktors, unter optionaler Zuführung der Reaktionsmischung aus j), gefahren. Die Umsetzung findet in Abwesenheit oder Gegenwart von Katalysatoren bei Reaktionstemperaturen von 140 bis 270 °C, vorzugsweise 160 bis 250 °C und unter einem Druck, der 5 bis 20 bar, vorzugsweise 7 bis 15 bar beträgt, innerhalb von 2 bis 20 Stunden, vorzugsweise 8 bis 15 Stunden, statt. Die kontinuierliche Austreibung des freigesetzten Ammoniaks wird unterstützt durch Alkoholbrüden, die in den Sumpf des Druckdestillationsreaktors in einer Menge von 0,5 bis 8 kg/kg, vorzugsweise 1 bis 4 kg/kg gebildetem Biscarbamat eingebracht und zweckmäßigerweise in einem am Sumpf der Kolonnne angebrachten Verdampfer erzeugt werden.
c) Diese Verfahrensweise erfolgt ohne die Schritte a) und b) in einem einstufigen Verfahrensschritt. Zur Herstellung der Biscarbamate in der Reaktionsstufe c) werden die Diamine der Formel (II) mit unkonditioniertem Harnstoff und einem Alkohol der Formel (III), gegebenenfalls auch Mischungen solcher Alkohole, in einem Molverhältnis von 1 : 2,01 : 4,0 bis 1 : 2,2 : 10, vorzugsweise 1 : 2,02 : 6 bis 1 : 2,12 : 9, wobei der im Molverhältnis angegebene zweite Zahlenwert sich auf Mole Harnstoff bezieht, gegebenenfalls aber nicht vorzugsweise in Gegenwart von Dialkycarbonaten, Carbamidsäurealkylestern oder Mischungen aus Dialkylcarbonaten und Carbamidsäureestern in einer Menge von jeweils 1 - 10 Mol-% bezogen auf das Diamin, unter optionaler Zuführung der Reaktionsmischung aus j), in Abwesenheit oder Gegenwart von Katalysatoren bei Reaktionstemperaturen von 140 - 270 °C, vorzugsweise 160-250 °C und unter einem Druck, der in Abhängigkeit vom eingesetzten Alkohol zwischen 2 - 80 bar, vorzugsweise 7 - 15 bar beträgt, innerhalb von 2 bis 20 Stunden, vorzugsweise 4 - 9 Stunden, zur Reaktion gebracht. Die Umsetzung kann in einer kontinuierlich betriebenen Rührkesselkaskade, vorzugsweise aber in einem Druckdestillationsreaktor, erfolgen.
   Zur Erhöhung der Reaktionsgeschwindigkeit können die Biscarbamate in Gegenwart von Katalysatoren hergestellt werden. Als Katalysatoren eignen sich anorganische oder organische Verbindungen, die ein oder mehrere, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14th Edition, publiziert von Chemical Rubber Publishing Co. 2310 SuperiorAve. N.E. Cleveland, Ohio, enthalten, beispielsweise Halogenide wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocaramate. Beispielhaft genannt seinen die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen, Kobalt und Nickel. Als typische Katalysatoren seinen beispielhaft folgende Verbindungen genannt: Lithiumethanolat, Lithiumbutanolat, Natriummethanolat, Kalium-tert.-butanolat, Magnesiumethanolat, Calciummethanolat, Zinn-(II)-chlorid, Zinn-(IV)-chlorid, Bleiacetat, Aluminiumtrichlorid, Bismuttrichlorid, Kupfer-(II)-acetat, Kupfer-(II)-chlorid, Zinkchlorid, Zinkoctoat, Titantetrabutanolat, Vanadiumtrichlorid, Vanadiumacetylacetoat, Mangan-(II)-acetat, Eisen-(II)-acetat, Eisen-(III)-acetat, Eisenoxalat, Cobaltchlorid, Cobaltnaphthenat, Nickelchlorid, Nickelnaphthenat sowie deren Mischungen. Die Katalysatoren können gegebenenfalls auch in Form ihrer Hydrate oder Ammoniakate zu Einsatz kommen.
   Ausgangsverbindungen für das erfindungsgemäße Verfahren sind Diamine der bereits obengenannten Formel (II), Alkohole der bereits obengenannten Formel (III) sowie unkonditionierter Harnstoff.
   Geeignete Diamine der Formel (II) sind aliphatische wie beispielsweise Hexamethylendiamin, 2-Methylpentamethylendiamin, Octamethylendiamin, 2,2,4- und 2,4,4-Trimethylhexamethylendiamin oder Gemische davon, Decamethylendiamin, 2-Methylnonamethylendiamin, Dodecamethylendiamin und cycloaliphatische Diamine, wie z. B. 1,4-Cyclohexandiamin, 1,3- oder 1,4-Cyclohexandimethanamin, 5-Amino-1,3,3-trimethylcyclohexanmethanamin (Isophorondiamin), 4,4'-Methylendicyclohexyldiamin, 2,4-Methylendicyclohexyldiamin, 2,2'-Methylendicyclohexyl-diamin und isomere (cyclo)aliphatische Diamine sowie perhydriertes Methylendiphenyldiamin. Methylendiphenyldiamin (MDA) fällt herstellungsbedingt als Isomerenmischung aus 4,4'-, 2,4- und 2,2'-MDA an (s. z. B. DE 101 27 273). Perhydriertes Methylendiphenyldiamin wird durch vollständige Hydrierung von MDA erhalten und ist demzufolge eine Mischung aus isomeren Methylendicyclohexyldiaminen (H₁₂MDA), nämlich 4,4'-, 2,4- und 2,2'-H₁₂MDA und eventuell geringen Mengen an nicht vollständig umgesetzten (teil)aromatischen MDA. Bevorzugt werden als Diamine der Formel (II) 5-Amino-1,3,3-trimethylcyclohexanmethanamin (Isophorondiamin), 2,2,4- und 2,4,4-Trimethylhexamethylendiamin oder Gemische daraus, 4,4'-Methylendicyclohexyldiamin, 2,4-Methylendicyclohexyldiamin und 2,2'-Methylendicyclohexyldiamin und auch beliebige Mischungen mindestens zweier dieser Isomere sowie Hexamethylendiamin und 2-Methylpentamethylendiamin eingesetzt.
   Als Alkohole der Formel (III) eignen sich beliebige aliphatische oder cycloaliphatische Alkohole, die unter Normaldruck einen unterhalb 190 °C liegenden Siedepunkt aufweisen. Beispielhaft genannt seinen C1-C6-Alkanole wie z. B. Methanol, Ethanol, 1-Propanol, 1-Butanol, 2-Butanol, 1-Hexanol oder Cyclohexanol. Bevorzugt wird 1-Butanol als Alkohol verwendet.
   Im Zuge der Umsetzung des Reaktionsgemisches wird Ammoniak freigesetzt, dessen Entfernung aus dem Reaktionsgleichgewicht sich als vorteilhaft erwiesen hat. Beim Austrag des Ammoniaks aus dem Reaktor ist darauf zu achten, dass die Wandtemperaturen des Reaktors und des Austragsrohres oberhalb von 60°C liegen, damit eine Belegung durch Ammoniumcarbaminat, welches in minimalen Mengen aus Ammoniak und Kohlendioxid durch Zersetzung von Harnstoff gebildet wird, vermieden werden kann. Es hat sich beispielsweise bewährt, die Umsetzung in einem Druckdestillationsreaktor durchzuführen, wobei das Reaktionsgemisch im Gegenstrom zu im Sumpf eingebrachten Alkoholbrüden geführt wird und auf diese Weise eine derartig intensive Durchmischung der Flüssigkeit auf den Böden, die praktisch jeweils einer Kaskadenstufe entsprechen, erfolgt.
d) Der überschüssige Alkohol, die Dialkylcarbonate, sofern solche gebildet wurden, oder Carbamidsäurealkylester oder Mischungen aus mindestens zwei dieser Komponenten werden einstufig, oder vorteilhafterweise zweistufig abgetrennt. Auf der ersten Stufe wird die Reaktionsmischung vom Druckniveau der Reaktionsstufe b) oder optional c) auf einen Druck von 1 bis 500 mbar, vorzugsweise 2 bis 150 mbar, entspannt und auf diese Weise in gasförmige Brüden, die die überwiegende Alkoholmenge sowie gegebenenfalls Dialkylcarbonate und/oder Carbamidsäurealkylester enthalten, und in einen flüssigen Austrag aufgetrennt. Im zweiten Schritt wird der flüssige Austrag durch Dünnschichtverdampfung bei 180 bis 250 °C, vorzugsweise 200 bis 230 °C, und einem Druck von 0,1 bis 20 mbar, vorzugsweise 1 bis 10 mbar, von gegebenenfalls vorhandenem restlichen Butanol sowie Mittelsiedern wie Dialkylcarbonaten und/oder Carbamidsäurealkylestern befreit, so dass der Rückstand im wesentlichen aus dem monomeren Biscarbamat und gegebenenfalls hochsiedenden Oligomeren besteht. Die Brüden können nach weiterer destillativer Reinigung in die Reaktionsstufe a), b) oder optional c) zurückgeführt werden. Eine Rückführung der Dialkylcarbonate und/oder Carbamidsäurealkylester in die Reaktionsstufe b) oder optional c) ist möglich aber nicht erforderlich.
e) Das am Kopf des Druckdestillationsreaktors aus b) oder optional c) abgezogene, dampfförmige Gemisch aus Alkohol und Ammoniak wird, ohne es zu kondensieren, vorzugsweise unter dem Druck des Druckdestillationsreaktors in den mittleren Bereich einer Destillationskolonne geführt, in der durch Rektifikation im Sumpf bei mindestens 170 °C, je nach gewähltem Alkohol und Betriebsdruck, ammoniakfreier Alkohol gewonnen wird, der in den Sumpf des Destillations- a) und des Druckdestillationsreaktors b) oder optional c) zurückgeführt wird. Am Kopf wird das Ammoniak flüssig abgezogen. Um die Belegung des Rückflusskondensators durch gegebenenfalls vorhandenes Ammoniumcarbamat zu verhindern, lässt man zur Temperaturerhöhung am Kopf auf mindestens 60°C einen entsprechenden Anteil an Alkohol zu. Die so mit dem Ammoniak aus dem Kreislauf ausgetragene Alkoholmenge muss durch Frischalkohol ersetzt werden.
f) Bevorzugt wird der nach Abtrennung von Leicht- und Mittelsiedern erhaltene flüssige, die monomeren Biscarbamate, und gegebenenfalls hochsiedende Oligomere enthaltende Stoffstrom aus Schritt b) oder optional c), vorzugsweise mit Hilfe eines Dünnschicht- oder Kurzwegverdampfers, bei einer Temperatur von 180 bis 270 °C, vorzugsweise 200 bis 250 °C und unter einem Druck von 0,01 bis 10 mbar, vorzugsweise 0,02 bis 5 mbar destillativ in einen Wertstoffstrom, der die monomeren Biscarbamate und die leichter siedenden Nebenprodukte enthält, und einen nicht destillierbaren Nebenproduktstrom getrennt werden. Der die hochsiedenden Komponenten enthaltende, nicht destillierbare Nebenproduktstrom wird aus dem Herstellverfahren ausgeschleust und üblicherweise als stofflich nicht verwertbarer Rückstand verworfen.
   Optional kann der gegebenenfalls hochsiedende Oligomere enthaltende Stoffstrom aus Stufe b) oder optional c) vor seiner oben beschriebenen destillativen Aufreinigung auch in zwei Teilströme aufgeteilt werden, von denen einer direkt der Deblockierungsreaktion (siehe g)) zugeführt wird und der andere zunächst die soeben beschriebene Hochsiederabtrennung durchläuft.
   Optional kann auf die Abtrennung der in der Reaktionsmischung aus Stufe d) gegebenenfalls enthaltenen Hochsieder verzichtet werden sofern die unter k) beschriebene Auftrennung des Recarbamatisatstroms aus Stufe j) durchgeführt wird.
g) Der die monomeren Biscarbamate und die leichter siedenden Nebenprodukte enthaltende Wertstoffstrom aus Stufe d) und optional aus Stufe f) wird in einer geeigneten Vorrichtung teilweise, lösemittelfrei in flüssiger Phase in Gegenwart von Katalysatoren bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch gespalten. Der Umsatz von Biscarbamat zu Diisocyanat in der Vorrichtung zur thermischen Spaltung kann in Abhängigkeit vom verwendeten Biscarbamat weitgehend frei gewählt werden und liegt üblicherweise in einem Bereich von 10 bis 95 Gew.-%, vorzugsweise 35 bis 85 Gew.-% der zugeführten Biscarbamatmenge (Feed). Der ungespaltende Anteil der Reaktionsmischung, der nicht umgesetzte Biscarbamate, hochsiedende Nebenprodukte und andere wieder verwertbare und unverwertbare Nebenprodukte enthält, wird kontinuierlich ausgeschleust. Die Menge der Ausschleusung richtet sich u. a. nach dem gewünschten Umsatz und der gewünschten Kapazität der Spaltreaktion und kann leicht experimentell ermittelt werden. Sie beträgt üblicherweise 10 bis 60 Gew.-%, vorzugsweise 15 bis 45 Gew.-%, bezogen auf den Feed.
   Als Katalysatoren zur chemischen Spaltung der Biscarbamate finden z. B. die vorgenannten, die Carbamatbildung katalysierenden anorganischen und organischen Verbindungen Verwendung. Vorzugsweise werden Chloride des Zinks, Zinns oder Kupfers sowie Zink-, Mangan-, Eisen-, oder Cobaltoxide eingesetzt, wobei der Katalysator dem Massenstrom aus der Stufe f) oder optional c) oder optional d) vor dessen Zuführung in die Spaltung als 0,01 bis 25 Gew.-%ige, vorzugsweise 0,05 bis 10 Gew.-%ige Lösung oder Suspension in dem Alkohol, der auch zur Carbamatherstellung verwendet wird, in einer Menge von 5 bis 400 ppm, vorzugsweise 10 bis 100 ppm, zudosiert wird.
   Als Spaltvorrichtungen eigenen sich beispielsweise zylinderförmige Spaltreaktoren, wie z. B. Röhrenöfen oder vorzugsweise Verdampfer, beispielsweise Fallfilm-, Dünnschicht- oder Bulkverdampfer, wie z. B. Robertverdampfer, Herbertverdampfer, caddle-typ-Verdampfer, Oskarverdampfer und Heizkerzenverdampfer.
   Bevorzugt wird die Spaltung in einer kombinierten Spalt- und Rektifizierkolonne durchgeführt, die für die Energiezufuhr im Sumpf mit einem Fallfilmverdampfer, im unteren Drittel mit einer Einrichtung zum zusätzlichen Energieeintrag bzw. zur Energierückgewinnung, im oberen Drittel mit einer Einrichtung zum Abzug von vorzugsweise Roh-Diisocyanat und am Kopf mit einem Kondensator, Kondensatsammelgefäß und einer Pumpe für den Rückfluss und den Abzug von reinem Alkohol ausgestattet ist.
   Aufgrund der Reaktivität der Isocyanatgruppen sollte deren mittlere Verweilzeit in der Spaltzone möglichst gering sein, was durch Minimierung des Flüssigkeitsvolumens durch entsprechende konstruktive Maßnahmen und durch Verwendung von geordneten Packungen mit geringem ,hold up' sowie durch möglichst unverzügliche destillative Entfernung des gebildeten Diisocyanats aus der Spaltzone erreicht wird. Letzteres wird durch entsprechenden Energieeintrag im Sumpf der kombinierten Spalt- und Rektifizierkolonne realisiert. Dadurch stellt sich ein Konzentrationsprofil in der Kolonne ein, wo im Sumpf im wesentlichen Biscarbamat, weniger als 10 Gew.-%, vorzugsweise weniger als 3 Gew.-% Diisocyanat und nicht nachweisbare Mengen an Alkohol vorhanden sind, während die Flüssigkeit im unteren Teil der Kolonne nur noch geringe Mengen an Biscarbamat, dagegen im wesentlichen aus Monoisocyanatomonocarbamat besteht. Der dazu erforderliche Rücklauf wird zweckmäßigerweise durch eine Köndensationstufe oberhalb der Spaltzone und unterhalb der des Diisocyanatseitenabzugs erzeugt. Diese Betriebsweise ist besonders wirtschaftlich, da die hier abzuführende Energie auf relativ hohem Temperaturniveau anfällt und anschließend nochmals genutzt werden kann z. B. zur Aufwärmung anderer Produktströme. Außerdem wird dadurch die Brüdenmenge entsprechend verringert, so dass oberhalb dieses Partialkondensators der Durchmesser der Kolonne entsprechend reduziert werden kann.
   Trotz der möglichst unverzüglichen destillativen Entfernung des gebildeten Diisocyanats aus der Spaltzone lässt sich die Bildung von höhermolekularen Verbindungen nicht restlos verhindern, so dass kontinuierlich ein dementsprechender Anteil aus dem Sumpf der kombinierten Spalt- und Rektifizierkolonne ausgeschleust und weiterbehandelt j) werden muss.
   Kurz zusammengefasst geht es in der Stufe der thermischen Spaltung der Biscarbamate prinzipiell darum, die mittlere Verweilzeit der Isocyanatgruppen, die bei Deblockierung des Alkohols zwangsläufig freigesetzt werden, in der Spaltzone möglichst gering zu halten und so unerwünschte Nebenreaktionen auf ein Minimum zu beschränken.
h) Die bei der thermischen Spaltung gebildeten Spaltprodukte, die sich vor allem aus Alkohol, Diisocyanat und partiell gespaltenen Biscarbamaten zusammensetzen, werden durch Rektifikation bei Temperaturen von 95 bis 260 °C, vorzugsweise 110 bis 245 °C und einem Druck von 0,5 bis 250 mbar, vorzugsweise 1 bis 100 mbar, in Alkohol und in eine rohe Diisocyanatmischung, bestehend aus (cyclo)aliphatischem Diisocyanat, partiell gespaltenem (cyclo)aliphatischem Biscarbamat, dem Monoisocyanatomonocarbamat, und gegebenenfalls geringen Anteilen an (cyclo)aliphatischen Biscarbamat, getrennt. Diese Trennung kann beispielsweise in der obengenannten kombinierten Spalt- und Rektifizierkolonne durchgeführt werden.
i) Die vorzugsweise durch Rektifikation erhaltene rohe Mischung bestehend aus (cyclo)aliphatischem Diisocyanat, partiell gespaltenem (cyclo)aliphatischem Biscarbamat, dem Monoisocyanatomonocarbamat, und gegebenenfalls geringen Anteilen an (cyclo)aliphatischem Biscarbamat, wird durch Destillation bei einer Temperatur von 95 bis 260 °C, vorzugsweise 110 bis 245 °C und unter einem Druck von 0,5 bis 150 mbar, vorzugsweise 1 bis 75 mbar, gereinigt, wobei die anfallenden Fraktionen zurückgeführt oder als Reinprodukt isoliert werden.
j) Die Sumpfausschleusung aus der Deblockierungsstufe g) wird partiell oder vollständig mit dem Alkohol aus der Rektifikationsstufe h) zusammengeführt, wobei das Molverhältnis von NCO-Gruppen und OH-Gruppen bis zu 1 : 100, vorzugsweise 1 : 20 und besonders bevorzugt 1 : 10 beträgt, und die Reaktionsmischung in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt. Die Umsetzung kann in einer kontinuierlichen Kesselkaskade oder in einem Rohrreaktor durchgeführt werden. Als Katalysatoren kommen grundsätzlich alle Kontakte in Frage, die die NCO/OH-Reaktion fördern. Beispielhaft seien Zinnoctoat, Dibutylzinnlaurat, Zinndichlorid, Zinkdichlorid, Kupferchlorid, Kupferdichlorid, Eisendichlorid, Eisentrichlorid und Triethylamin aufgeführt.
k) Der Reurethanisatstrom aus Stufe j) wird in einen Wertstoff- und einen Abfallstrom aufgetrennt und der an Hochsiederkomponenten reiche Abfallstrom aus dem Prozess ausgeschleust und verworfen. Die Auftrennung der beiden Stoffströme erfolgt vorzugsweise destillativ mit Hilfe eines Dünnschicht- oder Kurzwegverdampfers, bei einer Temperatur von 180 bis 270 °C, vorzugsweise 200 bis 250 °C und unter einem Druck von 0,01 bis 10 mbar, vorzugsweise 0,02 bis 5 mbar. Der Wertstoffstrom, der die monomeren Carbamate und die leichter siedenden Nebenprodukte enthält, fällt als Destillat an. Der an hochsiedenden Komponenten reiche Abfallstrom fällt als Rückstand an und wird aus dem Herstellverfahren ausgeschleust und üblicherweise als stofflich nicht verwertbares Material verworfen. Alternativ, aber nicht bevorzugt, kann die Auftrennung in Wert- und Abfallstoff auch durch Extraktion erfolgen. Als Extraktionsmittel ist beispielsweise überkritisches Kohlendioxid geeignet.
   Optional kann der Reurethanisatstrom aus Stufe j) vor der oben beschriebenen destillativen Aufreinigung auch in zwei Teilströme aufgeteilt werden, von denen einer direkt der Reinigungsstufe d) zugeführt wird. Die Aufteilung der beiden Ströme kann im Verhältnis 99 : 1 bis 1 : 99, bevorzugt 99 : 5 bis 5 : 95 erfolgen. Optional kann der zur Hochsiederabtrennung führende Reurethanisatstrom zunächst partiell oder vollständig von überschüssigem Alkohol befreit werden. Dies erfolgt vorzugsweise destillativ. Der abgetrennte Alkohol kann wahlweise in Stufe a), und/oder b) und/oder c) und/oder bevorzugt Stufe d) zurückgeführt werden.
l) Der Reurethanisatstrom aus Stufe j) kann vor einer destillativen Aufreinigung auch direkt in die (Cyclo)alkylenbiscarbamatstufe b) oder optional auch c) zugeführt werden.
m) Ein Teil der Sumpffraktion der Reindestillation i) wird kontinuierlich ausgeschleust und optional in die Deblockierungsstufe g) oder bevorzugt in die Carbamatisierungsstufe j) zurückgeführt. Die Menge der Ausschleusung beträgt 0,1 bis 50 Gew.-%, vorzugsweise 0,2 bis 25 Gew.-% des Zulaufs an Rohdiisocyanat in die Reindestillationsstufe.
n) Die Kopffraktion der Reindestillationstufe i) kann verworfen oder vorzugsweise in die Carbamatisierungsstufe j) zurückgeführt werden. Die Menge der pro Zeiteinheit abgeführten Kopffraktion beträgt 0,1 bis 3 Gew.-%, vorzugsweise 0,3 bis 1 Gew.-% des Zulaufs an Rohdiisocyanat in die Reindestillation.
o) Der aufgereinigte Reurethanisatstrom aus Stufe k) wird in die (Cyclo)alkylenbiscarbamatstufe b) oder optional auch c) und/oder in die Leicht- und Mittelsiederabtrennung d) und/oder in die Carbamatspaltung g) zurückgeführt.

Mit dem erfindungsgemäßen mehrstufigen Verfahren zur kontinuierlichen Herstellung von (cyclo)aliphatischen Diisocyanaten durch Umsetzung von (cyclo)aliphatischen Diaminen mit unkonditioniertem (unbehandeltem) Harnstoff und Alkoholen zu (cyclo)aliphatischen Biscarbamaten und anschließender thermischer Spaltung der (cyclo)aliphatischen Biscarbamate zu (cyclo)aliphatischen Diisocyanaten können (cyclo)aliphatische Diisocyanate mit hohen Reinheiten bei selektiv und störungsfrei ablaufenden Reaktionen produziert werden. Das erfindungsgemäße Verfahren eignet sich insbesondere zur Herstellung von aliphatischen Diisocyanaten mit einem geradkettigen oder verzweigten aliphatischen Kohlenwasserstoffrest mit insgesamt 6 bis 12 Kohlenstoffatomen wie Hexamethylendiisocyanat, 2-Methylpentandiisocyanat, 2,2,4- und 2,4,4-Trimethylhexamethylendiisocyanat oder Gemische davon, Octamethylendiisocyanat, Decamethylendiisocyanat, 2-Methylnonamethylendiisocyanat, Dodecamethylendiisocyanat oder einen gegebenenfalls substituierten cycloaliphatischen Kohlenwasserstoffrest mit insgesamt 4 bis 18, vorzugsweise 5 bis 15 Kohlenstoffatomen wie z.B. 1,4-Diisocyanatocyclohexan, 1,3- oder 1,4-Cyclohexandimethanisocyanat, 5-Isocyanato-1,3,3-trimethylcyclohexanmethanisocyanat (Isophorondiisocyanat) 4,4'-Methylendicyclohexyldiisocyanat (4,4'-H₁₂MDI), 2,2'-Methylendicyclohexyldiisocyanat (2,2'-H₁₂MDI), 2,4'-Methylendicyclohexyldiisocyanat (2,4'-H₁₂MDI) oder auch Mischungen der vorgenannten isomeren Methylendicyclohexyldiisocyanate (H₁₂MDI). Ganz besonders bevorzugt werden 5-Isocyanato-1,3,3-trimethylcyclohexanmethanisocyanat (Isophorondiisocyanat), 2,2,4- und 2,4,4-Trimethylhexa-methylendiisocyanat oder Gemische daraus und 4,4'-Methylendicyclohexyldiisocyanat sowie beliebige Mischungen aus 4,4'-H₁₂MDI, 2,4-H₁₂MDI und 2,2'-H₁₂MDI hergestellt.

Die hergestellten (cyclo)aliphatischen Diisocyanate eigenen sich sehr gut zur Produktion von Urethan-, Isocyanurat-, Amid- und/oder Harnstoffgruppen enthaltenden Kunststoffen nach dem Polyisocyanat-Polyadditionsverfahren. Sie finden darüber hinaus Verwendung zur Herstellung von mit Urethan-, Biuret-, und/oder Isocyanuratgruppen modifizierten Polyisocyanatmischungen. Derartige Polyisocyanatmischungen aus (cyclo)aliphatischen Diisocyanaten werden insbesondere zur Herstellung von hochwertigen, lichtbeständigen Polyurethanbeschichtungen eingesetzt.

Gegenstand der Erfindung sind auch Formaldehyd freie (cyclo)aliphatische Diisocyanate erhältlich durch Verfahren zur kontinuierlichen Herstellung von (cyclo)aliphatischen Diisocyanaten durch Umsetzung von mindestens einem (cyclo)aliphatischen Diamin mit Harnstoff und mindestens einem Alkohol zu (cyclo)aliphatischen Biscarbamaten und anschließender thermischer Spaltung der (cyclo)aliphatischen Biscarbamate zu (cyclo)aliphatischen Diisocyanaten dadurch gekennzeichnet, dass zur Bildung der (cyclo)aliphatischen Biscarbamate unkonditionierter Harnstoff eingesetzt wird, insbesondere Formaldehyd freie (cyclo)aliphatische Diisocyanate ausgewählt aus Isophorondiisocyanat, Trimethylhexamethylendiisocyanat, H₁₂MDI und Hexamethylendiisocyanat (HDI).

Formaldehyd frei bedeutet, dass die maximale Formaldehydkonzentration (auch Paraformaldehyd) des eingesetzten Harnstoffs 0,01 bis 0,10 Gew.-%, vorzugsweise 0,001 bis 0,01 Gew.-% und besonders bevorzugt weniger als 0,001 Gew.-% beträgt.

Die Erfindung wird durch die folgenden Beispiele näher erläutert.

Die Beispiele 1 bis 4 beschreiben die Herstellung von (cyclo)aliphatischen Diisocyanaten unter Einsatz von unkonditioniertem Harnstoff mit einem Formaldehydgehalt von < 10ppm. Die Vergleichsbeispiele A bis D beschreiben jeweils die Herstellung von (cyclo)aliphatischen Diisocyanaten unter Einsatz von handelsüblichem Harnstoff mit einem Formaldehydgehalt von 0,55 Gew.-%.

### Beispiel 1

Auf den ersten Boden eines Destillationsreaktors wurde stündlich ein Gemisch aus 41,0 kg 5-Amino-1,3,3-trimethylcyclohexanmethanamin, 29,8 kg unkonditionierten Harnstoff und 107,0 kg n-Butanol über einen mit Dampf beheizten Vorwärmer gepumpt. Aus der Reaktionsmischung wurde kontinuierlich das freigesetzte Ammoniak unter Normaldruck entfernt.

Die mittlere Verweilzeit im Destillationsreaktor betrug 7 h. Im Sumpf des unter Normaldruck betriebenen Destillationsreaktors wurden 12,5 kg/h Butanol aus dem Sumpf einer Ammoniak-Butanol-Trennkolonne in den Sumpf des Destillationsreaktors eingespeist. Die im Reboiler dem Destillationsreaktor zugeführte Energiemenge wird so eingeregelt, dass die am Kopf zusammen mit dem gebildeten Ammoniak anfallende und im Dephlegmator mit Warmwasser von 40 °C kondensierte Butanolmenge der im Sumpf eingebrachten entspricht. Der so kondensierte

Alkohol wird kontinuierlich in eine Ammoniak-Butanol-Trennkolonne gefahren.

Die im Sumpf des Destillationsreaktors anfallende Lösung von Bisharnstoff in Alkohol wurde standgeregelt über einen Vorwärmer, wo sie auf 190 bis 200 °C aufgeheizt wurde, zusammen mit 62,0 kg/h Reaktionsprodukt aus der Recarbamatisierungsstufe auf den obersten Boden des Druckdestillationsreaktors gefahren. Die mittlere Verweilzeit im Druckdestillationsreaktor betrug 10,5 h. Durch Heizung wurde folgendes Temperaturprofil eingestellt: Sumpf 229 °C und Kopf 200 °C. In den Sumpf des Druckdestillationsreaktors wurden 103,0 kg/h Butanol eingebracht und die Wärmeträgerölmenge zum Reboiler so eingeregelt, dass die am Kopf zusammen mit dem gebildeten Ammoniak abgezogene Butanolmenge der im Sumpf zugeführten entsprach.

Das erhaltene Butanol/Ammoniak-Gemisch wurde anschließend in die Ammoniak-Butanol-Trennkolonne gefahren. Die Kopftemperatur betrug dort 85 °C. Die Butanolverluste, die sich durch die Ammoniakausschleusung und aus sonstigen Verlusten (Leichtsiederkomponenten und der Verbrennung zugeführter Rückstände) ergaben, wurden durch Zufuhr von 4,7 kg/h Frischbutanol in dem Sumpf der Ammoniak-Butanol-Trennkolonne ersetzt. Das im Sumpf des Druckdestillationsreaktors anfallende Gemisch von stündlich 233,2 kg wurde durch Destillation gereinigt.

Die Einspeisung von 115,5 kg/h Biscarbamat auf den Fallfilmverdampfer der kombinierten Spalt- und Rektifizierkolonne erfolgte nach Zusatz von 0,2 kg/h Katalysator-Lsg. Die für die Spaltung und Rektifikation erforderliche Energie wurde mit Wärmeträgeröl im Fallfilmverdampfer übertragen. Die Carbamatspaltreaktion wurde bei einem Sumpfdruck von 27 mbar und einer Sumpftemperatur von 230 °C vorgenommen. Das während der Spaltung gebildete, durch Rektifikation am Kopf anfallende Butanol von 40,0 kg/h wurde abgezogen und mit der Sumpfausschleusung von stündlich 21,7 kg aus der kombinierten Spalt- und Rektifizierkolonne der Recarbamatisierungsstufe zugeführt.

Das aus der kombinierten Spalt- und Rektifizierkolonne im Seitenstrom abgezogene Roh-Diisocyanat von 55,4 kg/h wurde einer weiteren Reindestillation zugeführt und so 52,0 kg/h gereinigtes Diisocyanat erhalten. Die Reinheit des gewonnenen Diisocyanats wurde gaschromatografisch mit > 99,5 Gew.-% ermittelt. Die Gesamtverfahrensausbeute bezogen auf eingesetztes Diamin betrug 97,2 %.

### Vergleichsbeispiel A

Auf den ersten Boden eines Destillationsreaktors wurde stündlich ein Gemisch aus 35,7 kg 5-Amino-1,3,3-trimethylcyclohexanmethanamin, 25,9 kg konditionierter Harnstoff mit einem Formaldehydgehalt von 0,55 Gew.-% und 93,2 kg n-Butanol über einen mit Dampf beheizten Vorwärmer gepumpt. Aus der Reaktionsmischung wurde kontinuierlich das freigesetzte Ammoniak unter Normaldruck entfernt.

Die mittlere Verweilzeit im Destillationsreaktor betrug 7 h. Im Sumpf des unter Normaldruck betriebenen Destillationsreaktors wurden 11,0 kg/h Butanol aus dem Sumpf einer Ammoniak-Butanol-Trennkolonne in den Sumpf des Destillationsreaktors eingespeist. Die im Reboiler dem Destillationsreaktor zugeführte Energiemenge wird so eingeregelt, dass die am Kopf zusammen mit dem gebildeten Ammoniak anfallende und im Dephlegmator mit Warmwasser von 40 °C kondensierte Butanolmenge der im Sumpf eingebrachten entspricht. Der so kondensierte Alkohol wird kontinuierlich in eine Ammoniak-Butanol-Trennkolonne gefahren. Die im Sumpf des Destillationsreaktors anfallende Lösung von Bisharnstoff in Alkohol wurde standgeregelt über einen Vorwärmer, wo sie auf 190 bis 200 °C aufgeheizt wurde, zusammen mit 51,2 kg/h Reaktionsprodukt aus der Recarbamatisierungsstufe auf den obersten Boden des Druckdestillationsreaktors gefahren. Die mittlere Verweilzeit im Druckdestillationsreaktor betrug 10,5 h. Durch Heizung wurde folgendes Temperaturprofil eingestellt: Sumpf 229 °C und Kopf 200 °C. In den Sumpf des Druckdestillationsreaktors wurden 89,0 kg/h Butanol eingebracht und die Wärmeträgerölmenge zum Reboiler so eingeregelt, dass die am Kopf zusammen mit dem gebildeten Ammoniak abgezogene Butanolmenge der im Sumpf zugeführten entsprach.

Das erhaltene Butanol/Ammoniak-Gemisch wurde anschließend in die Ammoniak-Butanol-Trennkolonne gefahren. Die Kopftemperatur betrug dort 85 °C. Die Butanolverluste, die sich durch die Ammoniakausschleusung und aus sonstigen Verlusten (Leichtsiederkomponenten und der Verbrennung zugeführter Rückstände) ergaben, wurden durch Zufuhr von 4,5 kg/h Frischbutanol in dem Sumpf der Ammoniak-Butanol-Trennkolonne ersetzt. Das im Sumpf des Druckdestillationsreaktors anfallende Gemisch von 212,1 kg/h wurde durch Destillation gereinigt.

Die Einspeisung von stündlich 94,1 kg/h Biscarbamat auf den Fallfilmverdampfer der kombinierten Spalt- und Rektifizierkolonne erfolgte nach Zusatz von 0,2 kg/h Katalysator-Lsg.

Die für die Spaltung und Rektifikation erforderliche Energie wurde mit Wärmeträgeröl im Fallfilmverdampfer übertragen. Die Carbamatspaltreaktion wurde bei einem Sumpfdruck von 27 mbar und einer Sumpftemperatur von 234 °C vorgenommen. Das während der Spaltung gebildete, durch Rektifikation am Kopf anfallende Butanol 33,1 kg/h wurde abgezogen und mit der Sumpfausschleusung von 17,9 kg/h aus der kombinierten Spalt- und Rektifizierkolonne der Recarbamatisierungsstufe zugeführt.

Das aus der kombinierten Spalt- und Rektifizierkolonne im Seitenstrom abgezogene Roh-Diisocyanat von 45,82 kg/h wurde einer weiteren Reindestillation zugeführt und so 43,0 kg/h gereinigtes Diisocyanat erhalten. Die Reinheit des gewonnenen Diisocyanats wurde gaschromatografisch mit 98,6 Gew.-% ermittelt. Die Gesamtverfahrensausbeute bezogen auf eingesetztes Diamin betrug 92,3 %.

### Beispiel 2

Auf den ersten Boden eines Druckdestillationsreaktors wurde stündlich ein Gemisch aus 38,4 kg 5-Amino-1,3,3-trimethylcyclohexanmethanamin, 27,9 kg unkonditionierten Harnstoff, 100,1 kg n-Butanol und 57,4 kg Reaktionsprodukt aus der Recarbamatisierungsstufe über einen mit Dampf beheizten Vorwärmer, wo sie auf 190 bis 200°C aufgeheizt wurde, gepumpt.

Die mittlere Verweilzeit im Druckdestillationsreaktor betrug 10,5 h. Durch Heizung wurde folgendes Temperaturprofil eingestellt: Sumpf 230 °C und Kopf 200 °C. In den Sumpf des Druckdestillationsreaktors wurden 96,7 kg/h Butanol eingebracht und die Wärmeträgerölmenge zum Reboiler so eingeregelt, dass die am Kopf zusammen mit dem gebildeten Ammoniak abgezogene Butanolmenge der im Sumpf zugeführten entsprach.

Das erhaltene Butanol/Ammoniak-Gemisch wurde anschließend in die Ammoniak-Butanol-Trennkolonne gefahren. Die Kopftemperatur betrug dort 87 °C. Die Butanolverluste, die sich durch die Ammoniakausschleusung und aus sonstigen Verlusten (Leichtsiederkomponenten und der Verbrennung zugeführter Rückstände) ergaben, wurden durch Zufuhr von 4,7 kg/h Frischbutanol in dem Sumpf der Ammoniak-Butanol-Trennkolonne ersetzt. Das im Sumpf des Druckdestillationsreaktors anfallende Gemisch von 220,2 kg/h wurde durch Destillation gereinigt.

Die Einspeisung von 105,5 kg/h Biscarbamat auf den Fallfilmverdampfer der kombinierten

Spalt- und Rektifizierkolonne erfolgte nach Zusatz von 0,2 kg/h Katalysatör-Lsg. Die für die Spaltung und Rektifikation erforderliche Energie wurde mit Wärmeträgeröl im Fallfilmverdampfer übertragen. Die Carbamatspaltreaktion wurde bei einem Sumpfdruck von 27 mbar und einer Sumpftemperatur von 230 °C vorgenommen. Das während der Spaltung gebildete, durch Rektifikation am Kopf anfallende Butanol von 37,1 kg/h wurde abgezogen und mit der Sumpfausschleusung von stündlich 20,1 kg aus der kombinierten Spalt- und Rektifizierkolonne der Recarbamatisierungsstufe zugeführt.

Das aus der kombinierten Spalt- und Rektifizierkolonne im Seitenstrom abgezogene RohDiisocyanat von 51,4 kg/h wurde einer weiteren Reindestillation zugeführt und so 48,2 kg/h gereinigtes Diisocyanat erhalten. Die Reinheit des gewonnenen Diisocyanats wurde gaschromatografisch mit > 99,5 Gew.-% ermittelt. Die Gesamtverfahrensausbeute bezogen auf eingesetztes Diamin betrug 96,3 %.

### Vergleichsbeispiel B

Auf den ersten Boden eines Druckdestillationsreaktors wurde stündlich ein Gemisch aus 35,6 kg 5-Amino-1,3,3-trimethylcyclohexanmethanamin, 25,9 kg konditionierter Harnstoff mit einem Formaldehydgehalt von 0,55 Gew.-%, 93,0 kg n-Butanol und 50,9 kg Reaktionsprodukt aus der Recarbamatisierungsstufe über einen mit Dampf beheizten Vorwärmer, wo sie auf 190 bis 200 °C aufgeheizt wurde, gepumpt.

Die mittlere Verweilzeit im Druckdestillationsreaktor betrug 10,5 h. Durch Heizung wurde folgendes Temperaturprofil eingestellt: Sumpf 232 °C und Kopf 200 °C. In den Sumpf des Druckdestillationsreaktors wurden 88,9 kg/h Butanol eingebracht und die Wärmeträgerölmenge zum Reboiler so eingeregelt, dass die am Kopf zusammen mit dem gebildeten Ammoniak abgezogene Butanolmenge der im Sumpf zugeführten entsprach.

Das erhaltene Butanol/Ammoniak-Gemisch wurde anschließend in die Ammoniak-Butanol-Trennkolonne gefahren. Die Kopftemperatur betrug dort 88 °C. Die Butanolverluste, die sich durch die Ammoniakausschleusung und aus sonstigen Verlusten (Leichtsiederkomponenten und der Verbrennung zugeführter Rückstände) ergaben, wurden durch Zufuhr von 4,6 kg/h Frischbutanol in dem Sumpf der Ammoniak-Butanol-Trennkolonne ersetzt. Das im Sumpf des Druckdestillationsreaktors anfallende Gemisch von 212,3 kg/h wurde durch Destillation gereinigt.

Die Einspeisung von 93,4 kg/h Biscarbamat auf den Fallfilmverdampfer der kombinierten Spalt- und Rektifizierkolonne erfolgte nach Zusatz von 0,2 kg/h Katalysator-Lsg. Die für die Spaltung und Rektifikation erforderliche Energie wurde mit Wärmeträgeröl im Fallfilmverdampfer übertragen. Die Carbamatspaltreaktion wurde bei einem Sumpfdruck von 27 mbar und einer Sumpftemperatur von 234 °C vorgenommen. Das während der Spaltung gebildete, durch Rektifikation am Kopf stündlich anfallende Butanol von 32,8 kg wurde abgezogen und mit der Sumpfausschleusung von 17,8 kg/h aus der kombinierten Spalt- und Rektifizierkolonne der Recarbamatisierungsstufe zugeführt.

Das aus der kombinierten Spalt- und Rektifizierkolonne im Seitenstrom abgezogene Roh-Diisocyanat von 45,5 kg/h wurde einer weiteren Reindestillation zugeführt und so 42,7 kg/h gereinigtes Diisocyanat erhalten. Die Reinheit des gewonnenen Diisocyanats wurde gaschromatografisch mit 98,5 Gew.-% ermittelt. Die Gesamtverfahrensausbeute bezogen auf eingesetztes Diamin betrug 91,9 %.

### Beispiel 3

Auf den ersten Boden eines Druckdestillationsreaktors wurde stündlich ein Gemisch aus 34,7 kg (2,2,4-)2,4,4-Trimethylhexamethylendiamin, 27,2 kg unkonditionierten Harnstoff, 97,8 kg n-Butanol und 64,0 kg Reaktionsprodukt aus der Recarbamatisierungsstufe über einen mit Dampf beheizten Vorwärmer, wo sie auf 190 bis 200 °C aufgeheizt wurde, gepumpt.

Die mittlere Verweilzeit im Druckdestillationsreaktor betrug 10,5 h. Durch Heizung wurde folgendes Temperaturprofil eingestellt: Sumpf 228 °C und Kopf 200 °C. In den Sumpf des Druckdestillationsreaktors wurden 94,3 kg/h Butanol eingebracht und die Wärmeträgerölmenge zum Reboiler so eingeregelt, dass die am Kopf zusammen mit dem gebildeten Ammoniak abgezogene Butanolmenge der im Sumpf zugeführten entsprach.

Das erhaltene Butanol/Ammoniak-Gemisch wurde anschließend in die Ammoniak-Butanol-Trennkolonne gefahren. Die Kopftemperatur betrug dort 86 °C. Die Butanolverluste, die sich durch die Ammoniakausschleusung und aus sonstigen Verlusten (Leichtsiederkomponenten und der Verbrennung zugeführter Rückstände) ergaben, wurden durch Zufuhr von 4,5 kg/h Frischbutanol in dem Sumpf der Ammoniak-Butanol-Trennkolonne ersetzt. Das im Sumpf des Druckdestillationsreaktors anfallende Gemisch von 219,0 kg/h wurde durch Destillation gereinigt.

Die Einspeisung von stündlich 108,4 kg Biscarbamat auf den Fallfilmverdampfer der kombinierten Spalt- und Rektifizierkolonne erfolgte nach Zusatz von 0,2 kg/h Katalysator-Lsg Die für die Spaltung und Rektifikation erforderliche Energie wurde mit Wärmeträgeröl im Fallfilmverdampfer übertragen. Die Carbamatspaltreaktion wurde bei einem Sumpfdruck von 27 mbar und einer Sumpftemperatur von 228 °C vorgenommen. Das während der Spaltung gebildete, durch Rektifikation am Kopf anfallende Butanol von 38,1 kg/h wurde abgezogen und mit der Sumpfausschleusung von 25,7 kg/h aus der kombinierten Spalt- und Rektifizierkolonne der Recarbamatisierungsstufe zugeführt.

Das aus der kombinierten Spalt- und Rektifizierkolonne im Seitenstrom abgezogene Roh-Diisocyanat von 47,5 kg/h wurde einer weiteren Reindestillation zugeführt und 44,6 kg/h gereinigtes Diisocyanat erhalten. Die Reinheit des gewonnenen Diisocyanats wurde gaschromatografisch mit > 99,5 Gew.-% ermittelt. Die Gesamtverfahrensausbeute bezogen auf eingesetztes Diamin betrug 96,6 %.

### Vergleichsbeispiel C

Auf den ersten Boden eines Druckdestillationsreaktors wurde stündlich ein Gemisch aus 34,6 kg (2,2,4-)2,4,4-Trimethylhexamethylendiamin, 27,0 kg konditionierter Harnstoff mit einem Formaldehydgehalt von 0,55 Gew.%, 97,3 kg n-Butanol und 59,8 kg Reaktionsprodukt aus der Recarbamatisierungsstufe über einen mit Dampf beheizten Vorwärmer, wo sie auf 190 bis 200 °C aufgeheizt wurde, gepumpt.

Die mittlere Verweilzeit im Druckdestillationsreaktor betrug 10,5 h. Durch Heizung wurde folgendes Temperaturprofil eingestellt: Sumpf 231 °C und Kopf 200 °C. In den Sumpf des Druckdestillationsreaktors wurden 93,1 kg/h Butanol eingebracht und die Wärmeträgerölmenge zum Reboiler so eingeregelt, dass die am Kopf zusammen mit dem gebildeten Ammoniak abgezogene Butanolmenge der im Sumpf zugeführten entsprach.

Das erhaltene Butanol/Ammoniak-Gemisch wurde anschließend in die Ammoniak-Butanol-Trennkolonne gefahren. Die Kopftemperatur betrug dort 89 °C. Die Butanolverluste, die sich durch die Ammoniakausschleusung und aus sonstigen Verlusten (Leichtsiederkomponenten und der Verbrennung zugeführter Rückstände) ergaben, wurden durch Zufuhr von 5,1 kg/h Frischbutanol in dem Sumpf der Ammoniak-Butanol-Trennkolonne ersetzt. Das im Sumpf des Druckdestillationsreaktors anfallende Gemisch von stündlich 228,1 kg wurde durch Destillation gereinigt. Die Einspeisung von 101,3 kg/h Biscarbamat auf den Fallfilmverdampfer der kombinierten Spalt- und Rektifizierkolonne erfolgte nach Zusatz von 0,2 kg/h Katalysator-Lsg. Die für die Spaltung und Rektifikation erforderliche Energie wurde mit Wärmeträgeröl im Fallfilmverdampfer übertragen. Die Carbamatspaltreaktion wurde bei einem Sumpfdruck von 27 mbar und einer Sumpftemperatur von 233 °C vorgenommen. Das während der Spaltung gebildete, durch Rektifikation am Kopf anfallende Butanol von 35,6 kg/h wurde abgezogen und mit der Sumpfausschleusung von 24,0 kg/h aus der kombinierten Spalt- und Rektifizierkolonne der Recarbamatisierungsstufe zugeführt. Das aus der kombinierten Spalt- und Rektifizierkolonne im Seitenstrom abgezogene Roh-Diisocyanat von 44,4 kg/h wurde einer weiteren Reindestillation zugeführt und 41,7 kg/h gereinigtes Diisocyanat erhalten. Die Reinheit des gewonnenen Diisocyanats wurde gaschromatografisch mit 98,2 Gew.-% ermittelt. Die Gesamtverfahrensausbeute bezogen auf eingesetztes Diamin betrug 90,8 %.

### Beispiel 4

Der oberste Boden eines Druckdestillationsreaktors wurde stündlich mit 31,9 kg H₁₂MDA, 18,7 kg unkonditionierten Harnstoff und 67,4 kg n-Butanol beaufschlagt und die Reaktionsmischung unter kontinuierlicher Entfernung des freigesetzten Ammoniaks bei 10 bar, 220 °C und mit einer mittleren Verweilzeit von 10,5 h umgesetzt. Im Sumpf des Druckdestillationsreaktors wurden 66,1 kg/h Butanol eingespeist und die zusammen mit dem freigesetzten Ammoniak am Kopf abgezogene Alkoholmenge so gewählt, dass sie dem Alkoholeintrag im Sumpf entsprach. Das erhaltene Butanol/Ammoniak-Gemisch wurde anschließend in die Ammoniak-Butanol-Trennkolonne gefahren. Die Kopftemperatur betrug dort 86 °C. Die Butanolverluste, die sich durch die Ammoniakausschleusung und aus sonstigen Verlusten (Leichtsiederkomponenten und der Verbrennung zugeführter Rückstände) ergaben, wurden durch Zufuhr von Frischbutanol in dem Sumpf der Ammoniak-Butanol-Trennkolonne ersetzt. Der Reaktoraustrag wurde, zusammen mit dem Wertstoffstrom aus der Hochsiederabtrennung destillativ von überschüssigem Butanol, Leicht- und Mittelsiedern befreit und die verbleibenden 89,9 kg/h Bis-(4-butoxycarbonylaminocyclohexyl)-methan (H₁₂MDU) als Schmelze (140 °C) in die Wälzung des Fallfilmverdampfers der Spalt- und Rektifizierkolonne gefahren, wobei die Deblockierungsreaktion bei einer Temperatur von 234 °C und einem Sumpfdruck von 8 mbar in Gegenwart eines Katalysators durchgeführt wurde. Das gewonnene Roh-H₁₂MDI wurde einer Reindestillation zugeführt, wobei 37,3 kg/h Rein-H₁₂MDI erhalten wurden. 26,3 kg/h Rohbutanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurde kontinuierlich ein Teilstrom aus dem Wälzkreislauf ausgeschleust und zusammen mit 2,2 kg/h Sumpfauskreisung aus der H₁₂MDI-Reindestillation sowie dem Kopfprodukt aus der Spalt- und Rektifizierkolonne vereinigt und reurethanisiert. Der Reurethanisatstrom wurde von überschüssigem Butanol befreit und destillativ in einen Hochsieder reichen Abfallstrom und einen Wertstoffstrom aufgetrennt. Die 28,8 kg/h Wertstoffstrom wurden zusammen mit dem Reaktoraustrag der Diurethanherstellung der Flashstufe zugeführt. Die Reinheit des gewonnenen Diisocyanats wurde gaschromatografisch mit >99,5 Gew.-% ermittelt. Die Gesamtverfahrensausbeute bezogen auf eingesetztes Diamin betrug 93,8 %.

### Vergleichsbeispiel D

Der oberste Boden eines Druckdestillationsreaktors wurde stündlich mit 29,9 kg H₁₂MDA, 17,6 kg konditioniertem Harnstoff mit einem Formaldehydgehalt von 0,55 Gew.%, und 63,3 kg n-Butanol beaufschlagt und die Reaktionsmischung unter kontinuierlicher Entfernung des freigesetzten Ammoniaks bei 10 bar, 220 °C und mit einer mittleren Verweilzeit von 10,5 h umgesetzt. Im Sumpf des Druckdestillationsreaktors wurden stündlich 63,2 kg Butanol eingespeist und die zusammen mit dem freigesetzten Ammoniak am Kopf abgezogene Alkoholmenge so gewählt, dass sie dem Alkoholeintrag im Sumpf entsprach. Das erhaltene Butanol/Ammoniak-Gemisch wurde anschließend in die Ammoniak-Butanol-Trennkolonne gefahren. Die Kopftemperatur betrug dort 88 °C. Die Butanolverluste die sich durch die Ammoniakausschleusung und aus sonstigen Verlusten (Leichtsiederkomponenten und der Verbrennung zugeführter Rückstände) ergaben, wurden durch Zufuhr von Frischbutanol in dem Sumpf der Ammoniak-Butanol-Trennkolonne ersetzt. Der Reaktoraustrag wurde, zusammen mit dem Wertstoffstrom aus der Hochsiederabtrennung destillativ von überschüssigem Butanol, Leicht- und Mittelsiedern befreit und die verbleibenden 78,6 kg/h Bis-(4-butoxycarbonylaminocyclohexyl)-methan (H₁₂MDU) als Schmelze (140 °C) in die Wälzung des Fallfilmverdampfers der Spalt- und Rektifizierkolonne gefahren, wobei die Deblockierungsreaktion bei einer Temperatur von 240 °C und einem Sumpfdruck von 11 mbar in Gegenwart eines Katalysators durchgeführt wurde. Das gewonnene Roh-H₁₂MDI wurde einer Reindestillation zugeführt, wobei 32,6 kg/h Rein-H₁₂MDI erhalten wurden. 23,0 kg/h Rohbutanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurde kontinuierlich ein Teilstrom aus dem Wälzkreislauf ausgeschleust und zusammen mit 2,0 kg/h Sumpfauskreisung aus der H₁₂MDI-Reindestillation sowie dem Kopfprodukt aus der Spalt- und Rektifizierkolonne vereinigt und reurethanisiert. Der Reurethanisatstrom wurde von überschüssigem Butanol befreit und destillativ in einen Hochsieder reichen Abfallstrom und einen Wertstoffstrom aufgetrennt. Die 21,1 kg/h Wertstoffstrom wurden zusammen mit dem Reaktoraustrag der Diurethanherstellung der Flashstufe zugeführt. Die Reinheit des gewonnenen Diisocyanats wurde gaschromatografisch mit 98,0 Gew.-% ermittelt. Die Gesamtverfahrensausbeute bezogen auf eingesetztes Diamin betrug 87,3 %.

Die nachfolgende Tabelle 1 zeigt noch einmal zusammenfassend die wesentlichen Merkmale der Beispiele 1 bis 8 mit den signifikanten Unterschieden der Diisocyanatreinheiten und der Verfahrensausbeuten in Abhängigkeit der eingesetzten Harnstoffqualität.

**Tabelle 1**

| Bezeichnung | Dimension | Beispiel | | | | | | | |
|---|---|---|---|---|---|---|---|---|---|
| | | 1 | A | 2 | B | 3 | C | 4 | D |
| Diamin | kg/h | IPD 41 | IPD 35,7 | IPD 38,4 | IPD 35,6 | TMD 34,7 | TMD 34,6 | H₁₂MDA 31,9 | H₁₂MDA 29,9 |
| Harnstoff mit <10 ppm Formaldehyd | kg/h | 29,8 | 0 | 27,9 | 0 | 27,2 | 0 | 18,7 | 0 |
| Harnstoff mit 0,55 Gew.-% Formaldehyd | kg/h | 0 | 25,9 | 0 | 25,9 | 0 | 27,0 | 0 | 17,6 |
| Diisocyanat | kg/h | 52,0 | 43,0 | 48,2 | 42,7 | 44,6 | 41,7 | 37,3 | 32,6 |
| Diisocyanat Reinheit | Gew.% | >99,5 | 98,6 | >99,5 | 98,5 | >99,5 | 98,2 | >99,5 | 98,0 |
| Verfahrensausbeute | % | 97,2 | 92,3 | 96,3 | 91,9 | 96,6 | 90,8 | 93,8 | 87,3 |

| | | | | | | | | | |
|---|---|---|---|---|---|---|---|---|---|
| IPD: 5-Amino-1,3,3-trimethylcyclohexanmethanamin TMD: (2,2,4-)2,4,4-Trimethylhexamethylendiamin H₁₂MDA: Mischung aus isomerem Methylendicyclohexyldiamin | | | | | | | | | |

Die Diisocyanat-Reinheiten wurden gaschromatografisch ermittelt:
Gerät: HP3/ Agilent GC 6890
Trennsäule: HP 5 /Agilent 30m×320µmx0,25µm nominal

Die Verfahrensausbeute errechnet sich aus erzeugtem Diisocyanat bezogen auf eingesetztes Diamin.

## Patentansprüche

1. Verfahren zur kontinuierlichen Herstellung von (cyclo)aliphatischen Diisocyanaten durch Umsetzung von mindestens einem (cyclo)aliphatischen Diamin mit Harnstoff und mindestens einem Alkohol zu (cyclo)aliphatischen Biscarbamaten und anschließender thermischer Spaltung der (cyclo)aliphatischen Biscarbamate zu (cyclo)aliphatischen Diisocyanaten **dadurch gekennzeichnet, dass** zur Bildung der (cyclo)aliphatischen Biscarbamate unkonditionierter Harnstoff eingesetzt wird,
wobei der Harnstoff weder mit anorganischen Substanzen oberflächenbehandelt sein darf, und/oder aus einer mit Formaldehyd oder auch Paraformaldehyd behandelten Harnstoffschmelze stammen darf und/oder mit Formaldehyd oder auch Paraformaldehyd oberflächenbehandelt sein darf.

2. Mehrstufiges Verfahren nach Anspruch 1, zur kontinuierlichen Herstellung von (cyclo)aliphatischen Diisocyanaten durch Umsetzung von mindestens einem (cyclo)aliphatischen Diamin mit Harnstoff und mindestens einem Alkohol zu (cyclo)aliphatischen Biscarbamaten und anschließender thermischer Spaltung der (cyclo)aliphatischen Biscarbamate zu (cyclo)aliphatischen Diisocyanaten **dadurch gekennzeichnet, dass** zur Bildung der (cyclo)aliphatischen Biscarbamate nach einstufigen, zweistufigen oder mehrstufigen Verfahren, unkonditionierter Harnstoff eingesetzt wird.

3. Verfahren nach Anspruch 1 oder 2,
wobei die maximale Formaldehydkonzentration des eingesetzten Harnstoffs 0,01 bis 0,10 Gew.-%, vorzugsweise 0,001 bis 0,01 Gew.-% und besonders bevorzugt weniger als 0,001 Gew.-% beträgt.

4. Verfahren nach mindestens einem der vorherigen Ansprüche,
**dadurch gekennzeichnet,**
**dass** der Harnstoff mit keiner anorganischen Substanz und/oder Formaldehyd oberflächenbehandelt ist.

5. Verfahren nach Anspruch 4,
**dadurch gekennzeichnet,**
**dass** der Harnstoff kein Talkum, Bentonite, Kieselgur, Diatomeen, Kaolin oder andere silikatische Stoffe enthält.

6. Verfahren nach mindestens einem der Ansprüche 1 bis 5, zur kontinuierlichen Herstellung von (cyclo)aliphatischen Diisocyanaten der Formel (I)
OCN-R-NCO
wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, durch Umsetzung (cyclo)aliphatischer Diamine mit unkonditioniertem Harnstoff und Alkoholen zu (cyclo)aliphatischen Biscarbamaten und deren thermische Spaltung, wobei
a) (cyclo)aliphatische Diamine der Formel (II)
H₂N-R-NH₂
wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, mit unkonditioniertem Harnstoff in Gegenwart von Alkohol der Formel (III)
R¹-OH
wobei R¹ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Dialkycarbonaten, Carbamidsäurealkylestern oder Mischungen aus Dialkylcarbonaten und
Carbamidsäureestern und in Abwesenheit oder Gegenwart von Katalysatoren zu (Cyclo)alkylenbisharnstoff der Formel (IV)
H₂N-OC-HN-R-NH-CO-NH₂
wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, in einem Destillationsreaktor, unter gleichzeitiger Abtrennung des entstehenden Ammoniaks, umgesetzt werden, wobei die Edukte kontinuierlich auf den obersten Boden aufgegeben werden und das gebildete Ammoniak durch Destillation mit Alkoholbrüden, die im Sumpf eingeführt werden, ausgetrieben wird;
b) in der zweiten Stufe die Umsetzung des aus der ersten Stufe a) anfallenden (Cyclo)alkylenbisharnstoffs mit dem in a) als Lösungsmittel eingesetzten Alkohol in einem Druckdestillationsreaktor, unter gleichzeitiger Abtrennung des entstehenden Ammoniaks, zum (Cyclo)alkylenbiscarbamat der Formel (V)
R¹O-OC-HN-R-NH-CO-OR¹
durchgeführt wird.

7. Verfahren nach mindestens einem der Ansprüche 1 bis 5 zur kontinuierlichen Herstellung von (cyclo)aliphatischen Diisocyanaten der Formel (I)
OCN-R-NCO
wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, durch Umsetzung (cyclo)aliphatischer Diamine mit unkonditioniertem Harnstoff und Alkoholen zu (cyclo)aliphatischen Biscarbamaten und deren thermische Spaltung, wobei
c) die Umsetzung (cyclo)aliphatischer Diamine der Formel (II)
H₂N-R-NH₂
mit unkonditioniertem Harnstoff in Gegenwart von Alkohol der Formel (III)
R¹-OH
in einem Druckdestillationsreaktor einstufig, unter gleichzeitiger Abtrennung des entstehenden Ammoniaks, zum (Cyclo)alkylenbiscarbamat der Formel (V)
R¹O-OC-HN-R-NH-CO-OR¹
durchgeführt wird.

8. Verfahren nach mindestens einem der vorherigen Ansprüche, wobei d) aus der erhaltenen Reaktionsmischung aus b) oder optional c) der Alkohol, die Dialkylcarbonate und/oder Carbamidsäurealkylester abgetrennt werden und man den Alkohol sowie optional, auch die Dialkylcarbonate und/oder Carbamidsäurealkylester, in die Reaktionsstufe a) oder b) oder optional c) zurückführt.

9. Verfahren nach mindestens einem der vorherigen Ansprüche, wobei e) die Abtrennung von Ammoniak aus den am Kopf des Druckdestillationsreaktors sowohl aus b) oder optional aus c) anfallenden Brüden und aus dem Alkohol, der durch partielle Kondensation der Brüden aus dem Destillationsreaktor a) oder optional c) gewonnen wird, in einer nachgeschalteten Kolonne zweckmäßigerweise unter dem Druck des Druckdestillationsreaktors durchführt, wobei der im Sumpf anfallende ammoniakfreie Alkohol in den Sumpf des Destillationsreaktors und/oder in den Sumpf des Druckdestillationsreaktors recycliert wird.

10. Verfahren nach mindestens einem der vorherigen Ansprüche, wobei f) das von Leichtsiedern abgereicherte Roh-(cyclo)alkylenbiscarbamat aus d) vollständig oder partiell von hochsiedenden Rückständen abgetrennt oder optional auf eine Rückstandsabtrennung verzichtet wird.

11. Verfahren nach mindestens einem der vorherigen Ansprüche, wobei g) die über die Schritte d) und optional aus f) vorbehandelte (Cyclo)alkylenbiscarbamate enthaltende Reaktionsmischung in Gegenwart eines Katalysators kontinuierlich und lösemittelfrei bei Temperaturen von 180 bis 280 °C, vorzugsweise 200 bis 260 °C, und unter einem Druck von 0,1 bis 200 mbar, vorzugsweise 0,2 bis 100 mbar thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig aus dem Sumpf ausgeschleust wird.

12. Verfahren nach mindestens einem der vorherigen Ansprüche, wobei h) die Spaltprodukte aus Schritt g) durch Rektifikation in ein (cyclo)aliphatisches Rohdiisocyanat und Alkohol getrennt werden.

13. Verfahren nach mindestens einem der vorherigen Ansprüche, wobei
i) das (cyclo)aliphatische Rohdiisocyanat durch Destillation gereinigt und die (cyclo)aliphatisches Reindiisocyanat enthaltende Fraktion isoliert wird.

14. Verfahren nach mindestens einem der vorherigen Ansprüche, wobei
j) die Sumpfausschleusung aus g) partiell oder vollständig mit dem Alkohol aus h) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird, wobei das Molverhältnis von NCO-Gruppen und OH-Gruppen bis zu 1 : 100, vorzugsweise 1 : 20 und besonders bevorzugt 1 : 10 beträgt.

15. Verfahren nach mindestens einem der vorherigen Ansprüche, wobei k) die Reaktionsmischung aus j) in einen Wertstoff- und einen Abfallstrom aufgetrennt und der an Hochsiederkomponenten reiche Abfallstrom aus dem Prozess ausgeschleust und verworfen wird.

16. Verfahren nach mindestens einem der vorherigen Ansprüche, wobei
I) die Reaktionsmischung aus j) direkt in die (Cyclo)alkylenbiscarbamatstufe b) oder optional c) recycliert wird.

17. Verfahren nach mindestens einem der vorherigen Ansprüche, wobei m) ein Teil der Sumpffraktion der Reindestillation i) kontinuierlich ausgeschleust und in die Spaltreaktion g) und/oder in die Carbamatisierungsstufe j) geführt wird.

18. Verfahren nach mindestens einem der vorherigen Ansprüche, wobei n) die bei der Reindestillation des (cyclo)aliphatischen Rohdiisocyanats anfallenden Kopffraktionen ebenfalls in die Carbamatisierungsstufe j) zurückgeführt werden.

19. Verfahren nach mindestens einem der vorherigen Ansprüche, wobei o) der Wertstoffstrom aus k) in Stufe b) oder optional c) und/oder d) und/oder g) zurückgeführt wird.

20. Mehrstufiges Verfahren nach mindestens einem der Ansprüche 1 bis 5, zur kontinuierlichen Herstellung von (cyclo)aliphatischen Diisocyanaten der Formel (I)
OCN-R-NCO
wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, durch Umsetzung (cyclo)aliphatischer Diamine mit unkonditioniertem Harnstoff und Alkoholen zu (cyclo)aliphatischen Biscarbamaten und deren thermische Spaltung, wobei
a) (cyclo)aliphatische Diamine der Formel (II)
H₂N-R-NH₂
wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, mit unkonditioniertem Harnstoff in Gegenwart von Alkohol der Formel (III)
R¹-OH
wobei R¹ für einen Rest steht, wie er nach Entfernung der Hydroxylgruppe aus einem primären oder sekundären (cyclo)aliphatischen Alkohol mit 3 bis 8 Kohlenstoffatomen verbleibt, in Abwesenheit oder Gegenwart von Dialkycarbonaten, Carbamidsäurealkylestern oder Mischungen aus Dialkylcarbonaten und Carbamidsäureestern und in Abwesenheit oder Gegenwart von Katalysatoren zu (Cyclo)alkylenbisharnstoff der Formel (IV)
- H₂N-OC-HN-R-NH-CO-NH₂
wobei R für einen geradkettigen oder verzweigten aliphatischen Kohlenwasserstoffrest mit insgesamt 6 bis 12 Kohlenstoffatomen oder einen gegebenenfalls substituierten cycloaliphatischen Kohlenwasserstoffrest mit insgesamt 4 bis 18, vorzugsweise 5 bis 15 Kohlenstoffatomen steht, in einem Destillationsreaktor, unter gleichzeitiger Abtrennung des entstehenden Ammoniaks, umgesetzt werden, wobei die Edukte kontinuierlich auf den obersten Boden aufgegeben werden und das gebildete Ammoniak durch Destillation mit Alkoholbrüden, die im Sumpf eingeführt werden, ausgetrieben wird;
b) in der zweiten Stufe die Umsetzung des aus der ersten Stufe a) anfallenden (Cyclo)alkylenbisharnstoffs mit dem in a) als Lösungsmittel eingesetzten Alkohol in einem Druckdestillationsreaktor, unter gleichzeitiger Abtrennung des entstehenden Ammoniaks, zum (Cyclo)alkylenbiscarbamat der Formel (V)
R¹O-OC-HN-R-NH-CO-OR¹
durchgeführt wird;
c) oder optional die Umsetzung (cyclo)aliphatischer Diamine der Formel (II)
H₂N-R-NH₂
mit unkonditioniertem Harnstoff in Gegenwart von Alkohol der Formel (III)
R¹-OH
in einem Druckdestillationsreaktor einstufig, unter gleichzeitiger Abtrennung des entstehenden Ammoniaks, zum (Cyclo)alkylenbiscarbamat der Formel (V)
R¹O-OC-HN-R-NH-CO-OR¹
durchgeführt wird ohne die Schritte a) und b) (R und R¹ entspricht der obigen Definition);
d) aus der erhaltenen Reaktionsmischung aus b) oder optional c) der Alkohol, die Dialkylcarbonate und/oder Carbamidsäurealkylester abgetrennt werden und man den Alkohol sowie optional, auch die Dialkylcarbonate und/oder Carbamidsäurealkylester, in die Reaktionsstufe a) oder b) oder optional c) zurückführt;
e) die Abtrennung von Ammoniak aus den am Kopf des Druckdestillationsreaktors sowohl aus b) als auch optional aus c) anfallenden Brüden und aus dem Alkohol, der durch partielle Kondensation der Brüden aus dem Destillationsreaktor a) oder optional c) gewonnen wird, in einer nachgeschalteten Kolonne zweckmäßigerweise unter dem Druck des Druckdestillationsreaktors durchführt, wobei der im Sumpf anfallende ammoniakfreie Alkohol in den Sumpf des Destillationsreaktors und/oder in den Sumpf des Druckdestillationsreaktors recycliert wird;
f) das von Leichtsiedern abgereicherte Roh-(cyclo)alkylenbiscarbamat aus d) vollständig oder partiell von hochsiedenden Rückständen abgetrennt oder optional auf eine Rückstandsabtrennung verzichtet wird;
g) die über die Schritte d) und optional aus f) vorbehandelte (Cyclo)alkylenbiscarbamate enthaltende Reaktionsmischung in Gegenwart eines Katalysators kontinuierlich und lösemittelfrei bei Temperaturen von 180 bis 280°C, vorzugsweise 200 bis 260 °C, und unter einem Druck von 0,1 bis 200 mbar, vorzugsweise 0,2 bis 100 mbar thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig aus dem Sumpf ausgeschleust wird;
h) die Spaltprodukte aus Schritt g) durch Rektifikation in ein (cyclo)aliphatisches Rohdiisocyanat und Alkohol getrennt werden;
i) das (cyclo)aliphatische Rohdiisocyanat durch Destillation gereinigt und die (cyclo)aliphatisches Reindiisocyanat enthaltende Fraktion isoliert wird;
j) die Sumpfausschleusung aus g) partiell oder vollständig mit dem Alkohol aus h) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird und das Molverhältnis von NCO-Gruppen und OH-Gruppen bis zu 1 : 100, vorzugsweise 1 : 20 und besonders bevorzugt 1 : 10 beträgt;
k) die Reaktionsmischung aus j) in einen Wertstoff- und einen Abfallstrom aufgetrennt und der an Hochsiederkomponenten reiche Abfallstrom aus dem Prozess ausgeschleust und verworfen wird;
l) optional die Reaktionsmischung aus j) direkt in die (Cyclo)alkylenbiscarbamatstufe b) oder optional c) recycliert wird;
m) ein Teil der Sumpffraktion der Reindestillation i) kontinuierlich ausgeschleust und in die Spaltreaktion g) und/oder in die Carbamatisierungsstufe j) geführt wird;
n) optional die bei der Reindestillation des (cyclo)aliphatischen Rohdiisocyanats anfallenden Kopffraktionen ebenfalls in die Carbamatisierungsstufe j) zurückgeführt werden;
o) der Wertstoffstrom aus k) in Stufe b) und/oder optional c) und/oder d) und/oder g) zurückgeführt wird.

21. Verfahren nach mindestens einem der vorherigen Ansprüche,
**dadurch gekennzeichnet,**
**dass** Hexamethylendiamin, 2-Methylpentamethylendiamin, Octamethylendiamin, 2,2,4- und 2,4,4-Trimethylhexamethylendiamin oder Gemische davon, Decamethylendiam, 2-Methylnonamethylendiam, Dodecamethylendiam eingesetzt werden.

22. Verfahren nach mindestens einem der vorherigen Ansprüche,
**dadurch gekennzeichnet,**
**dass** 1,4-Cyclohexandiamin, 1,3- oder 1,4-Cyclohexandimethanamin, 5-Amino-1,3,3-trimethylcyclohexanmethanamin (Isophorondiamin), 4,4'-Methylendicyclohexyldiamin, 2,4-Methylendicyclohexyldiamin, 2,2'-Methylendicyclohexyldiamin (H₁₂MDA) und isomere (cyclo)aliphatische Diamine sowie perhydriertes Methylendiphenyldiamin eingesetzt werden.

23. Verfahren nach mindestens einem der vorherigen Ansprüche,
**dadurch gekennzeichnet,**
**dass** eine Mischung aus den isomeren Methylendicyclohexyldiaminen (H₁₂MDA), enthaltend 4,4'-, 2,4- und 2,2'-H₁₂MDA eingesetzt wird.

24. Verfahren nach mindestens einem der vorherigen Ansprüche,
**dadurch gekennzeichnet,**
**dass** als Diamine der Formel (II) 5-Amino-1,3,3-trimethylcyclohexanmethanamin (Isophorondiamin), 2,2,4- und 2,4,4-Trimethylhexamethylendiamin oder Gemische daraus, 4,4'-Methylendicyclohexyldiamin, 2,4-Methylendicyclohexyldiamin und 2,2'-Methylendicyclohexyldiamin und auch beliebige Mischungen mindestens zweier dieser Isomere, Hexamethylendiamin und/oder 2-Methylpentamethylendiamin eingesetzt werden.

25. Verfahren nach mindestens einem der vorherigen Ansprüche,
**dadurch gekennzeichnet,**
**dass** als Alkohole Methanol, Ethanol, 1-Propanol, 1-Butanol, 2-Butanol, 1-Hexanol und/oder Cyclohexanol eingesetzt werden.

26. Verfahren nach mindestens einem der vorherigen Ansprüche zur Herstellung von Hexamethylendiisocyanat, 2-Methylpentandiisocyanat, 2,2,4- und 2,4,4-Trimethylhexamethylendiisocyanat oder Gemische davon, Octamethylendiisocyanat, Decamethylendiisocyanat, 2-Methylnonamethylendiisocyanat, Dodecamethylendiisocyanat.

27. Verfahren nach mindestens einem der vorherigen Ansprüche zur Herstellung von 1,4-Diisocyanatocyclohexan, 1,3- oder 1,4-Cyclohexandimethanisocyanat, 5-Isocyanato-1,3,3-trimethylcyclohexanmethanisocyanat (Isophorondiisocyanat), 4,4'-Methylendicyclohexyldiisocyanat (4,4'-H₁₂MDI), 2,2'-Methylendicyclohexyldiisocyanat (2,2'-H₁₂MDI), 2,4'-Methylendicyclohexyldiisocyanat (2,4'-H₁₂MDI) oder auch Mischungen der vorgenannten isomeren Methylendicyclohexyldiisocyanate (H₁₂MDI).

28. Verfahren nach mindestens einem der vorherigen Ansprüche zur Herstellung von Isophorondiisocyanat.

29. Verfahren nach mindestens einem der vorherigen Ansprüche zur Herstellung von 4,4'-Methylendicyclohexyldiisocyanat sowie beliebige Mischungen aus 4,4'-H₁₂MDI, 2,4-H₁₂MDI und 2,2'-H₁₂MDI.

30. Verfahren nach mindestens einem der vorherigen Ansprüche zur Herstellung von 2,2,4-und 2,4,4-Trimethylhexamethylendiisocyanat oder Gemische daraus.

31. Verfahren nach mindestens einem der vorherigen Ansprüche zur Herstellung von Hexamethylendiisocyanat.

## Claims

1. Process for continuously preparing (cyclo)aliphatic diisocyanates by reacting at least one (cyclo) aliphatic diamine with urea and at least one alcohol to give (cyclo)aliphatic biscarbamates and subsequently thermally cleaving the (cyclo)aliphatic biscarbamates to (cyclo)aliphatic diisocyanates, **characterized in that** the (cyclo)aliphatic biscarbamates are formed by using unconditioned urea,
wherein the urea must not have been surface treated with inorganic substances and/or must not originate from a urea melt treated with formaldehyde or else paraformaldehyde and/or must not have been surface treated with formaldehyde or else paraformaldehyde.

2. Multistage process according to Claim 1, for continuously preparing (cyclo)aliphatic diisocyanates by reacting at least one (cyclo) aliphatic diamine with urea and at least one alcohol to give (cyclo)aliphatic biscarbamates and subsequently thermally cleaving the (cyclo)aliphatic biscarbamates to (cyclo)aliphatic diisocyanates, **characterized in that** the (cyclo)aliphatic biscarbamates are formed by one-stage, two-stage or multistage processes by using unconditioned urea.

3. Process according to Claim 1 or 2,
wherein the maximum formaldehyde concentration of the urea used is 0.01 to 0.10% by weight, preferably 0.001 to 0.01% by weight and more preferably less than 0.001% by weight.

4. Process according to at least one of the preceding claims,
**characterized in that**
the urea has not been surface treated with any inorganic substance and/or formaldehyde.

5. Process according to Claim 4,
**characterized in that**
the urea does not contain any talc, bentonites, kieselguhr, diatoms, kaolin or other silicatic substances.

6. Process according to at least one of Claims 1 to 5 for continuously preparing (cyclo)aliphatic diisocyanates of the formula (I)
OCN-R-NCO
where R is a straight-chain or branched aliphatic hydrocarbon radical having a total of 6 to 12 carbon atoms or an optionally substituted cycloaliphatic hydrocarbon radical having a total of 4 to 18 and preferably 5 to 15 carbon atoms, by reacting (cyclo)aliphatic diamines with unconditioned urea and alcohols to give (cyclo)aliphatic biscarbamates and the thermal cleavage thereof, wherein
a) (cyclo)aliphatic diamines of the formula (II)
H₂N-R-NH₂
where R is a straight-chain or branched aliphatic hydrocarbon radical having a total of 6 to 12 carbon atoms or an optionally substituted cycloaliphatic hydrocarbon radical having a total of 4 to 18 and preferably 5 to 15 carbon atoms are reacted with unconditioned urea in the presence of alcohol of the formula (III)
R¹-OH
where R¹ is a radical as remains after removal of the hydroxyl group from a primary or secondary (cyclo)aliphatic alcohol having 3 to 8 carbon atoms, in the absence or presence of dialkyl carbonates, alkyl carbamates or mixtures of dialkyl carbonates and carbamic esters, and in the absence or presence of catalysts, to give (cyclo)alkylenebisurea of the formula (IV)
H₂N-OC-HN-R-NH-CO-NH₂
where R is a straight-chain or branched aliphatic hydrocarbon radical having a total of 6 to 12 carbon atoms or an optionally substituted cycloaliphatic hydrocarbon radical having a total of 4 to 18 and preferably 5 to 15 carbon atoms, in a distillation reactor, with simultaneous removal of the ammonia formed, the reactants being introduced continuously to the uppermost tray and the ammonia formed being driven out by distillation with alcohol vapors which are introduced in the bottom;
b) in the second stage, the reaction of the (cyclo)alkylenebisurea obtained from the first stage a) with the alcohol used as solvent in a) is performed in a pressure distillation reactor with simultaneous removal of the ammonia formed to give the (cyclo)alkylene biscarbamate of the formula (V)
R¹O-OC-HN-R-NH-CO-OR¹.

7. Process according to at least one of Claims 1 to 5 for continuously preparing (cyclo)aliphatic diisocyanates of the formula (I)
OCN-R-NCO
where R is a straight-chain or branched aliphatic hydrocarbon radical having a total of 6 to 12 carbon atoms or an optionally substituted cycloaliphatic hydrocarbon radical having a total of 4 to 18 and preferably 5 to 15 carbon atoms, by reacting (cyclo)aliphatic diamines with unconditioned urea and alcohols to give (cyclo)aliphatic biscarbamates and the thermal cleavage thereof, wherein
c) the reaction of (cyclo)aliphatic diamines of the formula (II)
H₂N-R-NH₂
with unconditioned urea in the presence of alcohol of the formula (III)
R¹-OH,
is performed in a pressure distillation reactor in one stage with simultaneous removal of the ammonia formed to give the (cyclo)alkylene biscarbamate of the formula (V)
R¹O-OC-HN-R-NH-CO-OR¹.

8. Process according to at least one of the preceding claims, wherein
d) the alcohol, the dialkyl carbonates and/or alkyl carbamates are removed from the reaction mixture obtained from b) or optionally c), and the alcohol and optionally also the dialkyl carbonates and/or alkyl carbamates are recycled into reaction stage a) or b) or optionally c).

9. Process according to at least one of the preceding claims, wherein
e) the removal of ammonia from the vapors obtained at the top of the pressure distillation reactor either from b) or optionally from c), and from the alcohol which is obtained by partial condensation of the vapors from the distillation reactor a) or optionally c), is performed in a downstream column, appropriately under the pressure of the pressure distillation reactor, the ammonia-free alcohol obtained in the bottom being recycled into the bottom of the distillation reactor and/or into the bottom of the pressure distillation reactor.

10. Process according to at least one of the preceding claims, wherein
f) the crude (cyclo)alkylene biscarbamate depleted of low boilers from d) is removed completely or partially from high-boiling residues, or a residue removal is optionally omitted.

11. Process according to at least one of the preceding claims, wherein
g) the reaction mixture which contains (cyclo)alkylene biscarbamates and has been pretreated by means of steps d) and optionally f) is cleaved thermally in the presence of a catalyst, continuously and without solvent, at temperatures of 180 to 280°C, preferably 200 to 260°C, and under a pressure of 0.1 to 200 mbar, preferably 0.2 to 100 mbar, in such a way that a portion of the reaction mixture of 10 to 60% by weight based on the feed, preferably 15 to 45% by weight based on the feed, is discharged continuously from the bottom.

12. Process according to at least one of the preceding claims, wherein
h) the cleavage products from step g) are separated by rectification into a (cyclo)aliphatic crude diisocyanate and alcohol.

13. Process according to at least one of the preceding claims, wherein
i) the (cyclo)aliphatic crude diisocyanate is purified by distillation and the fraction containing (cyclo)aliphatic pure diisocyanate is isolated.

14. Process according to at least one of the preceding claims, wherein
j) the bottoms discharge from g) is reacted partially or completely with the alcohol from h) in the presence or absence of catalysts within 1 to 150 min, preferably 3 to 60 min, at temperatures of 20 to 200°C, preferably 50 to 170°C, and at a pressure of 0.5 to 20 bar, preferably 1 to 15 bar, where the molar ratio of NCO groups and OH groups is up to 1:100, preferably 1:20 and more preferably 1:10.

15. Process according to at least one of the preceding claims, wherein
k) the reaction mixture from j) is separated into a material of value stream and a waste stream, and the waste stream which is rich in high boiler components is discharged from the process and discarded.

16. Process according to at least one of the preceding claims, wherein
1) the reaction mixture from j) is recycled directly into the (cyclo)alkylene biscarbamate stage b) or optionally c).

17. Process according to at least one of the preceding claims, wherein
m) a portion of the bottoms fraction of the purifying distillation i) is discharged continuously and conducted into the cleavage reaction g) and/or into the carbamatization stage j).

18. Process according to at least one of the preceding claims, wherein
n) the top fractions obtained in the purifying distillation of the (cyclo)aliphatic crude diisocyanate are likewise recycled into the carbamatization stage j).

19. Process according to at least one of the preceding claims, wherein
o) the material of value stream from k) is recycled into stage b) or optionally c) and/or d) and/or g).

20. Multistage process according to at least one of Claims 1 to 5, for continuously preparing (cyclo)aliphatic diisocyanates of the formula (I)
OCN-R-NCO
where R is a straight-chain or branched aliphatic hydrocarbon radical having a total of 6 to 12 carbon atoms or an optionally substituted cycloaliphatic hydrocarbon radical having a total of 4 to 18 and preferably 5 to 15 carbon atoms, by reacting (cyclo)aliphatic diamines with unconditioned urea and alcohols to give (cyclo)aliphatic biscarbamates and the thermal cleavage thereof, wherein
a) (cyclo)aliphatic diamines of the formula (II)
H₂N-R-NH₂
where R is a straight-chain or branched aliphatic hydrocarbon radical having a total of 6 to 12 carbon atoms or an optionally substituted cycloaliphatic hydrocarbon radical having a total of 4 to 18 and preferably 5 to 15 carbon atoms are reacted with unconditioned urea in the presence of alcohol of the formula (III)
R¹-OH
where R¹ is a radical as remains after removal of the hydroxyl group from a primary or secondary (cyclo)aliphatic alcohol having 3 to 8 carbon atoms, in the absence or presence of dialkyl carbonates, alkyl carbamates or mixtures of dialkyl carbonates and carbamic esters, and in the absence or presence of catalysts, to give (cyclo)alkylenebisurea of the formula (IV)
H₂N-OC-HN-R-NH-CO-NH₂
where R is a straight-chain or branched aliphatic hydrocarbon radical having a total of 6 to 12 carbon atoms or an optionally substituted cycloaliphatic hydrocarbon radical having a total of 4 to 18 and preferably 5 to 15 carbon atoms, in a distillation reactor, with simultaneous removal of the ammonia formed, the reactants being introduced continuously to the uppermost tray and the ammonia formed being driven out by distillation with alcohol vapors which are introduced in the bottom;
b) in the second stage, the reaction of the (cyclo)alkylenebisurea obtained from the first stage a) with the alcohol used as solvent in a) is performed in a pressure distillation reactor with simultaneous removal of the ammonia formed to give the (cyclo)alkylene biscarbamate of the formula (V)
R¹O-OC-HN-R-NH-CO-OR¹;
c) or optionally the reaction of (cyclo)aliphatic diamines of the formula (II)
H₂N-R-NH₂
with unconditioned urea in the presence of alcohol of the formula (III)
R¹-OH,
is performed in a pressure distillation reactor in one stage with simultaneous removal of the ammonia formed to give the (cyclo)alkylene biscarbamate of the formula (V)
R¹O-OC-HN-R-NH-CO-OR¹
without steps a) and b) (R and R¹ correspond to the above definition);
d) the alcohol, the dialkyl carbonates and/or alkyl carbamates are removed from the reaction mixture obtained from b) or optionally c), and the alcohol and optionally also the dialkyl carbonates and/or alkyl carbamates are recycled into reaction stage a) or b) or optionally c);
e) the removal of ammonia from the vapors obtained at the top of the pressure distillation reactor either from b) or optionally from c), and from the alcohol which is obtained by partial condensation of the vapors from the distillation reactor a) or optionally c), is performed in a downstream column, appropriately under the pressure of the pressure distillation reactor, the ammonia-free alcohol obtained in the bottom being recycled into the bottom of the distillation reactor and/or into the bottom of the pressure distillation reactor;
f) the crude (cyclo)alkylene biscarbamate depleted of low boilers from d) is removed completely or partially from high-boiling residues, or a residue removal is optionally omitted;
g) the reaction mixture which contains (cyclo)alkylene biscarbamates and has been pretreated by means of steps d) and optionally f) is cleaved thermally in the presence of a catalyst, continuously and without solvent, at temperatures of 180 to 280°C, preferably 200 to 260°C, and under a pressure of 0.1 to 200 mbar, preferably 0.2 to 100 mbar, in such a way that a portion of the reaction mixture of 10 to 60% by weight based on the feed, preferably 15 to 45% by weight based on the feed, is discharged continuously from the bottom;
h) the cleavage products from step g) are separated by rectification into a (cyclo)aliphatic crude diisocyanate and alcohol;
i) the (cyclo)aliphatic crude diisocyanate is purified by distillation and the fraction containing (cyclo)aliphatic pure diisocyanate is isolated;
j) the bottoms discharge from g) is reacted partially or completely with the alcohol from h) in the presence or absence of catalysts within 1 to 150 min, preferably 3 to 60 min, at temperatures of 20 to 200°C, preferably 50 to 170°C, and at a pressure of 0.5 to 20 bar, preferably 1 to 15 bar, where the molar ratio of NCO groups and OH groups is up to 1:100, preferably 1:20 and more preferably 1:10;
k) the reaction mixture from j) is separated into a material of value stream and a waste stream, and the waste stream which is rich in high boiler components is discharged from the process and discarded;
l) optionally the reaction mixture from j) is recycled directly into the (cyclo)alkylene biscarbamate stage b) or optionally c);
m) a portion of the bottoms fraction of the purifying distillation i) is discharged continuously and conducted into the cleavage reaction g) and/or into the carbamatization stage j);
n) optionally the top fractions obtained in the purifying distillation of the (cyclo)aliphatic crude diisocyanate are likewise recycled into the carbamatization stage j);
o) the material of value stream from k) is recycled into stage b) or optionally c) and/or d) and/or g).

21. Process according to at least one of the preceding claims,
**characterized in that**
hexamethylenediamine, 2-methylpentamethylene-diamine, octamethylenediamine, 2,2,4- and 2,4,4-trimethylhexamethylenediamine or mixtures thereof, decamethylenediamine, 2-methylnonamethylene-diamine, dodecamethylenediamine are used.

22. Process according to at least one of the preceding claims,
**characterized in that**
1,4-cyclohexanediamine, 1,3- or 1,4-cyclohexane-dimethanamine, 5-amino-1,3,3-trimethylcyclohexane-methanamine (isophoronediamine), 4,4'methylenedicyclohexyldiamine, 2,4-methylenedicyclohexyldiamine, 2,2'-methylenedicyclohexyldiamine (H₁₂MDA) and isomeric (cyclo)aliphatic diamines, and also perhydrogenated methylenediphenyldiamine, are used.

23. Process according to at least one of the preceding claims,
**characterized in that**
a mixture of the isomeric methylenedicyclohexyl-diamines (H₁₂MDA) containing 4,4'-, 2,4- and 2,2'-H₁₂MDA is used.

24. Process according to at least one of the preceding claims, **characterized in that**
the diamines of the formula (II) used are 5-amino-1,3,3-trimethylcyclohexanemethanamine (isophoronediamine), 2,2,4- and 2,4,4-trimethylhexamethylene-diamine or mixtures thereof, 4,4'-methylenedicyclohexyldiamine, 2,4-methylenedicyclohexyldiamine and 2,2'-methylenedicyclohexyldiamine, and also any desired mixtures of at least two of these isomers, hexamethylenediamine and/or 2-methyl-pentamethylenediamine.

25. Process according to at least one of the preceding claims,
**characterized in that**
the alcohols used are methanol, ethanol, 1-propanol, 1-butanol, 2-butanol, 1-hexanol and/or cyclohexanol.

26. Process according to at least one of the preceding claims for preparing hexamethylene diisocyanate, 2-methylpentane diisocyanate, 2,2,4- and 2,4,4-trimethylhexamethylene diisocyanate or mixtures thereof, octamethylene diisocyanate, decamethylene diisocyanate, 2-methylnonamethylene diisocyanate, dodecamethylene diisocyanate.

27. Process according to at least one of the preceding claims for preparing 1,4-diisocyanatocyclohexane, 1,3- or 1,4-cyclohexanedimethane isocyanate, 5-isocyanato-1,3,3-trimethylcyclohexanemethane isocyanate (isophorone diisocyanate), 4,4'-methylenedicyclohexyl diisocyanate (4,4'-H₁₂MDI), 2,2'-methylenedicyclohexyl diisocyanate (2,2'-H₁₂MDI), 2,4'-methylenedicyclohexyl diisocyanate (2,4'-H₁₂MDI), or else mixtures of the aforementioned isomeric methylenedicyclohexyl diisocyanates (H₁₂MDI).

28. Process according to at least one of the preceding claims for preparing isophorone diisocyanate.

29. Process according to at least one of the preceding claims for preparing 4,4'-methylenedicyclohexyl diisocyanate, and any desired mixtures of 4,4'-H₁₂MDI, 2,4-H₁₂MDI and 2,2-H₁₂MDI.

30. Process according to at least one of the preceding claims for preparing 2,2,4- and 2,4,4-trimethylhexamethylene diisocyanate or mixtures thereof.

31. Process according to at least one of the preceding claims for preparing hexamethylene diisocyanate.

## Revendications

1. Procédé pour la production continue de diisocyanates (cyclo)aliphatiques par mise en réaction d'au moins une diamine (cyclo)aliphatique avec de l'urée et au moins un alcool, conduisant à des biscarbamates (cyclo)aliphatiques, et coupure thermique subséquente des biscarbamates (cyclo)aliphatiques en diisocyanates (cyclo)aliphatiques, **caractérisé en ce que** pour la formation des biscarbamates (cyclo)aliphatiques on utilise de l'urée non conditionnée,
l'urée n'ayant pas à être traitée en surface par des substances inorganiques et/ou n'ayant pas à provenir d'une masse fondue d'urée traitée par du formaldéhyde ou bien du paraformaldéhyde et/ou n'ayant pas à être traitée en surface par du formaldéhyde ou bien du paraformaldéhyde.

2. Procédé en plusieurs étapes selon la revendication 1, pour la production continue de diisocyanates (cyclo)aliphatiques par mise en réaction d'au moins une diamine (cyclo)aliphatique avec de l'urée et au moins un alcool, conduisant à des biscarbamates (cyclo)aliphatiques, et coupure thermique subséquente des biscarbamates (cyclo)aliphatiques en diisocyanates (cyclo)aliphatiques, **caractérisé en ce que** pour la formation des biscarbamates (cyclo)aliphatiques selon des procédés en une étape, en deux étapes ou en plus de deux étapes on utilise de l'urée non conditionnée.

3. Procédé selon la revendication 1 ou 2,
dans lequel la teneur maximale en formaldéhyde de l'urée utilisée vaut 0,01 à 0,10 % en poids, de préférence 0,001 à 0,01 % en poids et de façon particulièrement préférée moins de 0,001 % en poids.

4. Procédé selon au moins l'une quelconque des revendications précédentes,
**caractérisé en ce que**
l'urée n'est traitée en surface par aucune substance inorganique et/ou aucun formaldéhyde.

5. Procédé selon la revendication 4,
**caractérisé en ce que**
l'urée ne contient pas de talc, de bentonites, de kieselguhr, de terre à diatomées, de kaolin ni d'autres matières de type silicate.

6. Procédé selon au moins l'une quelconque des revendications 1 à 5, pour la production continue de diisocyanates (cyclo)aliphatiques de formule (I)
OCN-R-NCO
dans laquelle R représente un radical hydrocarboné aliphatique à chaîne droite ou ramifié ayant au total de 6 à 12 atomes de carbone ou un radical hydrocarboné cycloaliphatique éventuellement substitué ayant au total de 4 à 18, de préférence de 5 à 15 atomes de carbone, par mise en réaction de diamines (cyclo)aliphatiques avec de l'urée non conditionnée et des alcools, conduisant à des biscarbamates (cyclo)aliphatiques, et coupure thermique de ces derniers, dans lequel
a) on fait réagir dans un réacteur de distillation des diamines (cyclo)aliphatiques de formule (II)
H₂N-R-NH₂
dans laquelle R représente un radical hydrocarboné aliphatique à chaîne droite ou ramifié ayant au total de 6 à 12 atomes de carbone ou un radical hydrocarboné cycloaliphatique éventuellement substitué ayant au total de 4 à 18, de préférence de 5 à 15 atomes de carbone, avec de l'urée non conditionnée, en présence d'un alcool de formule (III)
R¹OH
dans laquelle R¹ représente un radical, tel qu'il reste après élimination du groupe hydroxy d'un alcool (cyclo)aliphatique primaire ou secondaire ayant de 3 à 8 atomes de carbone, en absence ou en présence de carbonates de dialkyle, de carbamates d'alkyle ou de mélanges de carbonates de dialkyle et d'esters d'acide carbamique et en absence ou en présence de catalyseurs, pour l'obtention d'une (cyclo)alkylènebisurée de formule (IV)
H₂N-OC-HN-R-NH-CO-NH₂
dans laquelle R représente un radical hydrocarboné aliphatique à chaîne droite ou ramifié ayant au total de 6 à 12 atomes de carbone ou un radical hydrocarboné cycloaliphatique éventuellement substitué ayant au total de 4 à 18, de préférence de 5 à 15 atomes de carbone, avec séparation simultanée de l'ammoniac résultant, les produits de départ étant amenés en continu sur le plateau le plus élevé et l'ammoniac formé étant évacué par distillation avec des vapeurs d'alcool qui sont introduites dans le pied ;
b) dans la deuxième étape on effectue la réaction de la (cyclo)alkylènebisurée provenant de la première étape a) avec l'alcool utilisé comme solvant en a), dans un réacteur de distillation sous pression, avec séparation simultanée de l'ammoniac résultant, pour obtenir le (cyclo)alkylènebiscarbamate de formule (V)
R¹O-OC-HN-R-NH-CO-OR¹.

7. Procédé selon au moins l'une quelconque des revendications 1 à 5, pour la production continue de diisocyanates (cyclo)aliphatiques de formule (I)
OCN-R-NCO
dans laquelle R représente un radical hydrocarboné aliphatique à chaîne droite ou ramifié ayant au total de 6 à 12 atomes de carbone ou un radical hydrocarboné cycloaliphatique éventuellement substitué ayant au total de 4 à 18, de préférence de 5 à 15 atomes de carbone, par mise en réaction de diamines (cyclo)aliphatiques avec de l'urée non conditionnée et des alcools, conduisant à des biscarbamates (cyclo)aliphatiques, et coupure thermique de ces derniers, dans lequel
c) on effectue la réaction de diamines (cyclo)aliphatiques de formule (II)
H₂N-R-NH₂
avec de l'urée non conditionnée, en présence d'un alcool de formule (III)
R¹OH
en une étape dans un réacteur de distillation sous pression, avec séparation simultanée de l'ammoniac résultant, pour obtenir le (cyclo)alkylènebiscarbamate de formule (V)
R¹O-OC-HN-R-NH-CO-OR¹.

8. Procédé selon au moins l'une quelconque des revendications précédentes, dans lequel
d) on sépare du mélange réactionnel obtenu, provenant de b) ou en option c), l'alcool, les carbonates de dialkyle et/ou les carbamates d'alkyle et on recycle dans l'étage de réaction a) ou b) ou en option c) l'alcool ainsi qu'en option également les carbonates de dialkyle et/ou carbamates d'alkyle.

9. Procédé selon au moins l'une quelconque des revendications précédentes, dans lequel
e) on effectue dans une colonne raccordée en aval, convenablement sous la pression du réacteur de distillation sous pression, la séparation de l'ammoniac à partir des vapeurs apparaissant à la tête du réacteur de distillation aussi bien de b) qu'en option de c) et à partir de l'alcool qui est obtenu par condensation partielle des vapeurs provenant du réacteur de distillation a) ou en option c), l'alcool exempt d'ammoniac apparaissant dans le pied étant recyclé dans le pied du réacteur de distillation et/ou dans le pied du réacteur de distillation sous pression.

10. Procédé selon au moins l'une quelconque des revendications précédentes, dans lequel
f) on sépare totalement ou partiellement des résidus à haut point d'ébullition le (cyclo)alkylènebiscarbamate brut appauvri en composants à bas point d'ébullition, provenant de d), ou en option on se dispense d'une séparation des résidus.

11. Procédé selon au moins l'une quelconque des revendications précédentes, dans lequel
g) on soumet en continu à une coupure thermique le mélange réactionnel contenant des (cyclo)alkylène-biscarbamates, prétraité par les étapes d) et en option provenant de f), à des températures de 180 à 280 °C, de préférence de 200 à 260 °C, et sous une pression de 0,1 à 200 mbars, de préférence de 0,2 à 100 mbars en présence d'un catalyseur et sans solvant, de sorte qu'une partie du mélange réactionnel de 10 à 60 % en poids, par rapport à la charge d'alimentation, de préférence de 15 à 45 % en poids, par rapport à la charge d'alimentation, est déchargée en continu du pied.

12. Procédé selon au moins l'une quelconque des revendications précédentes, dans lequel
h) les produits de coupure provenant de l'étape g) sont fractionnés par rectification en un diisocyanate (cyclo)aliphatique brut et alcool.

13. Procédé selon au moins l'une quelconque des revendications précédentes, dans lequel
i) on purifie par distillation le diisocyanate (cyclo)aliphatique brut et on isole la fraction contenant du diisocyanate (cyclo)aliphatique pur.

14. Procédé selon au moins l'une quelconque des revendications précédentes, dans lequel
j) le courant de décharge de pied provenant de g) est mis partiellement ou totalement en réaction avec l'alcool provenant de h), en présence ou en absence de catalyseurs, en l'espace de 1 à 150 min, de préférence de 3 à 60 min, à des températures de 20 à 200 °C, de préférence de 50 à 170 °C et sous une pression de 0,5 à 20 bars, de préférence de 1 à 15 bars, le rapport molaire des groupes NCO et des groupes OH valant jusqu'à 1 : 100, de préférence 1 : 20 et de façon particulièrement préférée 1 : 10.

15. Procédé selon au moins l'une quelconque des revendications précédentes, dans lequel
k) le mélange réactionnel provenant de j) est fractionné en un courant de substance de valeur et un courant de déchets et le courant de déchets riche en composants à haut point d'ébullition est évacué du processus et rejeté.

16. Procédé selon au moins l'une quelconque des revendications précédentes, dans lequel
l) le mélange réactionnel provenant de j) est recyclé directement dans l'étage (cyclo)alkylènebiscarbamate b) ou en option c).

17. Procédé selon au moins l'une quelconque des revendications précédentes, dans lequel
m) une partie de la fraction de pied de la distillation conduisant à l'état pur i) est évacuée en continu et envoyée dans la réaction de coupure g) et/ou dans l'étage de carbamatation j).

18. Procédé selon au moins l'une quelconque des revendications précédentes, dans lequel
n) les fractions de tête apparaissant lors de la distillation conduisant à l'état pur du diisocyanate (cyclo)aliphatique brut sont également renvoyées dans l'étage de carbamatation j).

19. Procédé selon au moins l'une quelconque des revendications précédentes, dans lequel
o) le courant de substance de valeur provenant de k) est renvoyé dans l'étage b) ou en option c) et/ou d) et/ou g).

20. Procédé en plusieurs étapes selon au moins l'une quelconque des revendications 1 à 5, pour la production continue de diisocyanates (cyclo)-aliphatiques de formule (I)
OCN-R-NCO
dans laquelle R représente un radical hydrocarboné aliphatique à chaîne droite ou ramifié ayant au total de 6 à 12 atomes de carbone ou un radical hydrocarboné cycloaliphatique éventuellement substitué ayant au total de 4 à 18, de préférence de 5 à 15 atomes de carbone, par mise en réaction de diamines (cyclo)aliphatiques avec de l'urée non conditionnée et des alcools, conduisant à des biscarbamates (cyclo)aliphatiques, et coupure thermique de ces derniers, dans lequel
a) on fait réagir dans un réacteur de distillation des diamines (cyclo)aliphatiques de formule (II)
H₂N-R-NH₂
dans laquelle R représente un radical hydrocarboné aliphatique à chaîne droite ou ramifié ayant au total de 6 à 12 atomes de carbone ou un radical hydrocarboné cycloaliphatique éventuellement substitué ayant au total de 4 à 18, de préférence de 5 à 15 atomes de carbone, avec de l'urée non conditionnée, en présence d'un alcool de formule (III)
R¹OH
dans laquelle R¹ représente un radical, tel qu'il reste après élimination du groupe hydroxy d'un alcool (cyclo)aliphatique primaire ou secondaire ayant de 3 à 8 atomes de carbone, en absence ou en présence de carbonates de dialkyle, de carbamates d'alkyle ou de mélanges de carbonates de dialkyle et d'esters d'acide carbamique et en absence ou en présence de catalyseurs, pour l'obtention d'une (cyclo)alkylènebisurée de formule (IV)
H₂N-OC-HN-R-NH-CO-NH₂
dans laquelle R représente un radical hydrocarboné aliphatique à chaîne droite ou ramifié ayant au total de 6 à 12 atomes de carbone ou un radical hydrocarboné cycloaliphatique éventuellement substitué ayant au total de 4 à 18, de préférence de 5 à 15 atomes de carbone, avec séparation simultanée de l'ammoniac résultant, les produits de départ étant amenés en continu sur le plateau le plus élevé et l'ammoniac formé étant évacué par distillation avec des vapeurs d'alcool qui sont introduites dans le pied ;
b) dans la deuxième étape on effectue la réaction de la (cyclo)alkylènebisurée provenant de la première étape a) avec l'alcool utilisé comme solvant en a), dans un réacteur de distillation sous pression, avec séparation simultanée de l'ammoniac résultant, pour obtenir le (cyclo)alkylène-biscarbamate de formule (V)
R¹O-OC-HN-R-NH-CO-OR¹ ;
c) ou en option on effectue la réaction de diamines (cyclo)aliphatiques de formule (II)
H₂N-R-NH₂
avec de l'urée non conditionnée, en présence d'un alcool de formule (III)
R¹OH
en une étape dans un réacteur de distillation sous pression, avec séparation simultanée de l'ammoniac résultant, pour obtenir le (cyclo)alkylène-biscarbamate de formule (V)
R¹O-OC-HN-R-NH-CO-OR¹
sans les étapes a) et b) (R et R' correspondent à la définition donnée plus haut) ;
d) on sépare du mélange réactionnel obtenu, provenant de b) ou en option c), l'alcool, les carbonates de dialkyle et/ou les carbamates d'alkyle et on recycle dans l'étage de réaction a) ou b) ou en option c) l'alcool ainsi qu'en option également les carbonates de dialkyle et/ou carbamates d'alkyle ;
e) on effectue dans une colonne raccordée en aval, convenablement sous la pression du réacteur de distillation sous pression, la séparation de l'ammoniac à partir des vapeurs apparaissant à la tête du réacteur de distillation aussi bien de b) qu'en option de c) et à partir de l'alcool qui est obtenu par condensation partielle des vapeurs provenant du réacteur de distillation a) ou en option c), l'alcool exempt d'ammoniac apparaissant dans le pied étant recyclé dans le pied du réacteur de distillation et/ou dans le pied du réacteur de distillation sous pression ;
f) on sépare totalement ou partiellement des résidus à haut point d'ébullition le (cyclo)alkylène-biscarbamate brut appauvri en composants à bas point d'ébullition, provenant de d), ou en option on se dispense d'une séparation des résidus ;
g) on soumet en continu à une coupure thermique le mélange réactionnel contenant des (cyclo)alkylène-biscarbamates, prétraité par les étapes d) et en option provenant de f), à des températures de 180 à 280 °C, de préférence de 200 à 260 °C, et sous une pression de 0,1 à 200 mbars, de préférence de 0,2 à 100 mbars en présence d'un catalyseur et sans solvant, de sorte qu'une partie du mélange réactionnel de 10 à 60 % en poids, par rapport à la charge d'alimentation, de préférence de 15 à 45 % en poids, par rapport à la charge d'alimentation, est déchargée en continu du pied ;
h) les produits de coupure provenant de l'étape g) sont fractionnés par rectification en un diisocyanate (cyclo)aliphatique brut et alcool ;
i) on purifie par distillation le diisocyanate (cyclo)aliphatique brut et on isole la fraction contenant du diisocyanate (cyclo)aliphatique pur ;
j) le courant de décharge de pied provenant de g) est mis partiellement ou totalement en réaction avec l'alcool provenant de h), en présence ou en absence de catalyseurs, en l'espace de 1 à 150 min, de préférence de 3 à 60 min, à des températures de 20 à 200 °C, de préférence de 50 à 170 °C et sous une pression de 0,5 à 20 bars, de préférence de 1 à 15 bars, et le rapport molaire des groupes NCO et des groupes OH vaut jusqu'à 1 : 100, de préférence 1 : 20 et de façon particulièrement préférée 1 : 10 ;
k) le mélange réactionnel provenant de j) est fractionné en un courant de substance de valeur et un courant de déchets et le courant de déchets riche en composants à haut point d'ébullition est évacué du processus et rejeté ;
l) en option le mélange réactionnel provenant de j) est recyclé directement dans l'étage (cyclo)alkylènebiscarbamate b) ou en option c) ;
m) une partie de la fraction de pied de la distillation conduisant à l'état pur i) est évacuée en continu et envoyée dans la réaction de coupure g) et/ou dans l'étage de carbamatation j) ;
n) les fractions de tête apparaissant lors de la distillation conduisant à l'état pur du diisocyanate (cyclo)aliphatique brut sont également renvoyées dans l'étage de carbamatation j) ;
o) le courant de substance de valeur provenant de k) est renvoyé dans l'étage b) ou en option c) et/ou d) et/ou g).

21. Procédé selon au moins l'une quelconque des revendications précédentes,
**caractérisé en ce**
**qu'**on utilise l'hexaméthylènediamine, la 2-méthylpenta-méthylènediamine, l'octaméthylènediamine, la 2,2,4- et la 2,4,4-triméthylhexaméthylènediamine ou des mélanges de celles-ci, la décaméthylènediamine, la 2-méthylnonaméthylènediamine, la dodécaméthylènediamine.

22. Procédé selon au moins l'une quelconque des revendications précédentes,
**caractérisé en ce que**
qu'on utilise la 1,4-cyclohexanediamine, la 1,3- ou 1,4-cyclohexanediméthanamine, la 5-amino-1,3,3-triméthylcyclohexaneméthanamine (isphoronediamine), la 4,4'-méthylènedicyclohexyldiamine, la 2,4-méthylènedicyclohexyldiamine, la 2,2'-méthylènedicyclohexyl-diamine (H₁₂MDA) et des diamines (cyclo)aliphatiques isomères ainsi que la méthylènediphényldiamine perhydrogénée.

23. Procédé selon au moins l'une quelconque des revendications précédentes,
**caractérisé en ce**
**qu'**on utilise un mélange des méthylènedicyclohexyl-diamines isomères (H₁₂MDA) contenant de la 4,4'-, de la 2,4- et de la 2,2'-H₁₂MDA.

24. Procédé selon au moins l'une quelconque des revendications précédentes,
**caractérisé en ce**
**qu'**on utilise comme diamines de formule (II) la 5-amino-1,3,3-triméthylcyclohexaneméthanamine (isophoronediamine), la 2,2,4- et la 2,4,4-triméthylhexaméthylènediamine ou des mélanges de celles-ci, la 4,4'-méthylènedicyclohexyldiamine, la 2,4-méthylènedicyclohexyldiamine et la 2,2'-méthylènedicyclohexyl-diamine et également des mélanges quelconques de deux de ces isomères, l'hexaméthylènediamine et/ou la 2-méthylpentaméthylènediamine.

25. Procédé selon au moins l'une quelconque des revendications précédentes,
**caractérisé en ce**
**qu'**on utilise comme alcools le méthanol, l'éthanol, le 1-propanol, le 1-butanol, le 2-butanol, le 1-hexanol et/ou le cyclohexanol.

26. Procédé selon au moins l'une quelconque des revendications précédentes, pour la production d'hexaméthylènediisocyanate, de 2-méthylpentane-diisocyanate, de 2,2,4- et 2,4,4-triméthyl-hexaméthylènediisocyanate ou de mélanges de ceux-ci, d'octaméthylènediisocyanate, de décaméthylène-diisocyanate, de 2-méthylnonaméthylènediisocyanate, de dodécaméthylènediisocyanate.

27. Procédé selon au moins l'une quelconque des revendications précédentes pour la production de 1,4-diisocyanatocyclohexane, de 1,3 ou 1,4-cyclohexanediméthane-isocyanate, 5-isocyanato-1,3,3-triméthylcyclohexaneméthane-isocyanate (isophoronediisocyanate), 4,4'-méthylènedicyclohexyldiisocyanate (4,4'-H₁₂MDI), 2,2'-méthylènedicyclohexyldiisocyanate (2,2'-H₁₂MDI), 2,4'-méthylènedicyclohexyldiisocyanate (2,4'-H₁₂MDI) ou bien de mélanges des méthylènedicyclohexyldiisocyanates (H₁₂MDI) isomères précités.

28. Procédé selon au moins l'une quelconque des revendications précédentes, pour la production d'isophoronediisocyanate.

29. Procédé selon au moins l'une quelconque des revendications précédentes, pour la production de 4,4'-méthylènedicyclohexyldiisocyanate ainsi que de mélanges quelconques de 4,4'-H₁₂MDI, 2,4-H₁₂MDI et 2,2'-H₁₂MDI.

30. Procédé selon au moins l'une quelconque des revendications précédentes, pour la production de 2,2,4- et 2,4,4-triméthylhexaméthylènediisocyanate ou de mélanges de ceux-ci.

31. Procédé selon au moins l'une quelconque des revendications précédentes, pour la production d'hexaméthylènediisocyanate.
